# EUROPEAN PATENT APPLICATION

(11) **EP 4 039 330 A1**
(43) Date of publication of application: **10.08.2022**
(21) Application number: 21382560.7
(22) Date of filing: 25.06.2021
(51) Int. Cl.: A61P 25/00, A61P 35/00, A61P 35/04, C12Q 1/6886

(54) **METHODS AND COMPOSITIONS FOR THE TREATMENT OF NEUROBLASTOMA MALIGNANCIES**

(30) Priority: 04.02.2021 EP 21382092
(71) Applicant: Consejo Superior De Investigaciones Científicas (CSIC), 28006 Madrid (ES)
(72) Inventor: MARTI GOROSTIZA, Elisa, 08036 Barcelona (ES); LE DREAU, Gwenvael, 08036 Barcelona (ES); FANLO ESCUDERO, Lucia, 08036 Barcelona (ES)
(74) Representative: Pons

(57) **Abstract**

The present invention relates to an activator of a gene belonging to the SA-c1 module for use in the treatment of NB malignancies in a subject, as well as to an *in vitro* method for designing a personalized therapy for a subject with NB malignancies.

## Description

### FIELD OF THE INVENTION

The present invention relates to methods and compositions for use in the treatment and/or prevention of neuroblastoma malignancies. More specifically, it concerns the use of an activator of a gene belonging to the SA-c1 module in the treatment and/or prevention of neuroblastoma malignancies in a subject.

### BACKGROUND OF THE INVENTION

Neuroblastoma (NB) represents the most common cancer in infants and the most frequent solid extracranial tumour in childhood, accounting for 10-15% of cancer-related child deaths. NBs are nearly all sporadic (98%) and present a high degree of heterogeneity, which is reflected by the diversity of primary NB locations, with half of the tumours being detected in para-spinal ganglia in the abdomen, chest and neck, while the other half are found in the adrenal gland medulla. About 60% of NB tumours are classified as low-to-intermediate risk (LR-NBs) and correspond mostly to loco-regional tumours that present a good prognosis and can even regress spontaneously. By contrast, the remaining 40% of NB cases are classified as high-risk (HR-NBs) tumours, and they are often already metastatic at the time of diagnosis, displaying a strong resistance to therapy and a high probability of relapse that is reflected in poor patient outcome.

Treatment of NBs depends on their level of risk and includes for example surgery, radiotherapy, chemotherapy, combination of chemotherapy with monoclonal antibody therapy (dinutuximab) and targeted therapy. The most predictive events of a poor prognosis for NB patients appear to be amplification of the proto-oncogene MYCN, and mutations in the ALK, ATRX and PHOX2B genes. However, these genetic alterations are only found in up to 16% of all NB cases and are thus insufficient to explain the differences in the aetiology and malignancy of HR-NBs relative to LR-NBs.

NBs originate from alterations to cells in the sympatho-adrenal (SA) lineage, one of the multiple cell fate branches of neural crest cells. This SA lineage is composed of four cell identities: Schwann cell precursors, bridge cells, chromaffin cells and sympathoblasts. There is some debate regarding the specific identity of the NB cell-of-origin. One study argued that adrenal NBs derive from fetal chromaffin cells, and that LR-NBs and HR-NBs can be discriminated based on the degree of chromaffin cell differentation. Another study pinpointed instead fetal sympathoblasts as the NB cell-of-origin, yet could not discriminate NB subtypes based on this signature. Another study found that the signatures of sympathoblasts and norepinephrine-chromaffin cells were highly enriched in samples from NB patients, but suggested that the signature of Schwann cell precursors is the one distinguishing LR-NBs from HR-NBs.

Thus, more work is needed to elucidate whether LR-NBs and HR-NBs differ in the way they exploit the transcriptional program underlying their developmental SA origin, and more importantly, whether this SA program might be exploited to identify novel factors capable of impeding the metastatic dissemination and malignancy of HR-NBs, and to subsequently design novel, personalized therapies to impede said metastatic dissemination.

### DETAILED DESCRIPTION OF THE INVENTION

NB is a devastating paediatric cancer originating in the sympatho-adrenal lineage derived from the neural crest. Unlike LR-NBs, HR-NBs are metastatic and display a strong resistance to therapy and high rates of relapse, leading to poor prognosis. Identifying the molecular signatures that discriminate LR- from HR-NBs should thus facilitate the design of efficient therapies against aggressive NB tumours.

By comparing the transcriptional landscapes of LR- and HR-NBs with that of the healthy human fetal adrenal gland (a major site of NB formation), the inventors have identified the transcriptional signatures associated to the formation of both LR- and HR-NBs. Among these genes relevant to NB formation, the inventors have further identified 503 genes, organised into four different clusters (c1-c4), which are differentially expressed between LR-NBs and HR-NBs (LR *vs* HR DEGs). The main cluster c1 is particularly interesting in that it identified 338 genes that are expressed at significantly lower levels in HR-NBs than in LR-NBs, so that these c1 genes might impede the formation of metastases specifically observed in HR-NBs.

Furthermore, the authors have demonstrated that LR-NBs and HR-NBs can be discriminated by a transcriptional signature corresponding to the core SA lineage and consisting of 4,518 genes shared by at least 3 of the 4 cell types forming the human SA lineage. Remarkably, more than half of the 503 LR *vs* HR DEGs were retrieved in this core SA developmental signature. Even more strikingly, 92% of this core SA signature corresponded to genes belonging to the cluster c1 (242 genes out of 262, hereafter named "SA-c1" genes or SA-c1 module).

Thus, the inventors demonstrate here for the first time that the transcriptional landscapes of LR-NB and HR-NBs can be discriminated based on the SA-c1 signature corresponding to the core SA lineage, and that this developmental program is unexpectedly associated with a favourable prognosis. Indeed, said genes are strikingly predictive of NB malignancy, patient prognosis and disease progression. One of these genes, encoding the secreted *Neurexophilin-1* (NXPH-1) protein, and its receptor α-NRXN1, have been further characterized in functional assays. The results obtained confirmed the dual role of these two molecules; the interaction between the ligand NXPH1 and its receptor α-NRXN1 and the subsequent signalling cascade promote NB growth while unexpectedly inhibiting the formation of metastases.

The findings thereby uncover the genes comprising the SA-c1 module, and more specifically NXPH1/α-NRXN1 signalling, as possible therapeutic targets for the treatment of HR-NBs. Additionally, the findings shed a new light on the complex contribution of the neural crest-derived developmental program to NB pathogenesis and reveal that the developmental SA program appears to facilitate NB formation, but also to concomitantly restrict its malignant potential. The present invention thus provides activators of the genes comprising the SA-c1 module for use in the treatment of NB malignancies, particularly HR-NBs. The activators of the invention are particularly helpful for NB patients showing at least one metastasis, and for those patients affected by NB tumours displaying a strong resistance to conventional therapy.

In one aspect, the invention discloses an activator of a gene belonging to the SA-c1 module, or a pharmaceutical composition comprising said activator in a therapeutically effective amount, for use in the treatment of NB malignancies in a subject, wherein the gene is selected from the SA-c1 module listed in Table 1.

**Table 1. SA-c1 genes ranked by their fold change expression between LR-NBS and HR-NBs (highest #1 to lowest #242).**

| **Rank (risk disease)** | **Gene name (primary)** | **Cross-reference (GeneID)** | **UniProtKB** | **Entry name** |
|---|---|---|---|---|
| #01 | SOX6 | 55553 | P35712 | SOX6_HUMAN |
| #02 | NXPH1 | 30010 | P58417 | NXPH1_HUMAN |
| #03 | PRPH | 5630 | P41219 | PRPH_HUMAN |
| #04 | TFAP2B | 7021 | Q92481 | AP2B_HUMAN |
| #05 | EPHA7 | 2026 | P09104 | ENOG_HUMAN |
| #06 | SCN2A | 6326 | Q99250 | SCN2A_HUMAN |
| #07 | VSTM2A | 222008 | Q8TAG5 | VTM2A_HUMAN |
| #08 | SCN9A | 6335 | Q15858 | SCN9A_HUMAN |
| #09 | PLXNA4 | 91584 | Q9HCM2 | PLXA4_HUMAN |
| #10 | SCN3A | 6328 | Q9NY46 | SCN3A_HUMAN |
| #11 | TLN2 | 83660 | Q9Y4G6 | TLN2_HUMAN |
| #12 | CNTNAP2 | 1272 | Q12860 | CNTN1_HUMAN |
| #13 | ADCY1 | 107 | Q08828 | ADCY1_HUMAN |
| #14 | RGMB | 285704 | Q6NW40 | RGMB_HUMAN |
| #15 | PSD2 | 84249 | Q9BQ17 | PSD2_HUMAN |
| #16 | KIF5A | 3800 | O60282 | KIF5C_HUMAN |
| #17 | SCG2 | 7857 | P13521 | SCG2_HUMAN |
| #18 | ST8SIA2 | 8128 | Q92186 | SIA8B_HUMAN |
| #19 | CNTN1 | 1271 | P26992 | CNTFR_HUMAN |
| #20 | PRUNE2 | 158471 | Q8WUY3 | PRUN2_HUMAN |
| #21 | DLG2 | 54769 | Q96HU8 | DIRA2_HUMAN |
| #22 | CAMK2B | 816 | Q13554 | KCC2B_HUMAN |
| #23 | TMEM196 | 256130 | Q5HYL7 | TM196_HUMAN |
| #24 | PCDHB6 | 56130 | Q9Y5E3 | PCDB6_HUMAN |
| #25 | CAMTA1 | 23261 | Q9Y6Y1 | CMTA1_HUMAN |
| #26 | FAM134B | 26122 | Q52LR7 | EPC2_HUMAN |
| #27 | GRIA4 | 2893 | P48058 | GRIA4_HUMAN |
| #28 | AGPAT4 | 56895 | Q9NRZ5 | PLCD_HUMAN |
| #29 | PVRL1 | 22841 | Q7L804 | RFIP2_HUMAN |
| #30 | FRZB | 27086 | Q9H334 | FOXP1_HUMAN |
| #31 | PIK3R1 | 5295 | P27986 | P85A_HUMAN |
| #32 | NALCN | 4684 | P13591 | NCAM1_HUMAN |
| #33 | CORO2A | 26047 | Q9UHC6 | CNTP2_HUMAN |
| #34 | DPP6 | 23312 | Q8TDJ6 | DMXL2_HUMAN |
| #35 | SYT17 | 51760 | Q9BSW7 | SYT17_HUMAN |
| #36 | PTN | 5764 | P21246 | PTN_HUMAN |
| #37 | RIMS1 | 22999 | Q86UR5 | RIMS1_HUMAN |
| #38 | ZCCHC12 | 170261 | Q6PEW1 | ZCH12_HUMAN |
| #39 | GNAO1 | 2775 | P09471 | GNAO_HUMAN |
| #40 | SHANK2 | 22941 | Q9UPX8 | SHAN2_HUMAN |
| #41 | RGS7 | 6000 | P49802 | RGS7_HUMAN |
| #42 | TMOD1 | 7111 | P28289 | TMOD1_HUMAN |
| #43 | SCN3B | 55800 | Q9NY72 | SCN3B_HUMAN |
| #44 | UNC80 | 285175 | Q8N2C7 | UNC80_HUMAN |
| #45 | GABBR1 | 9846 | Q9UQC2 | GAB2_HUMAN |
| #46 | NCOA7 | 5818 | Q15223 | NECT1_HUMAN |
| #47 | GATA2 | 2550 | Q9UBS5 | GABR1_HUMAN |
| #48 | SRGAP3 | 9901 | O43295 | SRGP3_HUMAN |
| #49 | ICA1L | 130026 | Q8NDH6 | ICA1L_HUMAN |
| #50 | MYO5A | 4649 | B2RTY4 | MYO9A_HUMAN |
| #51 | LPAR1 | 26045 | O43300 | LRRT2_HUMAN |
| #52 | KCTD1 | 284252 | Q719H9 | KCTD1_HUMAN |
| #53 | SYT7 | 9066 | 043581 | SYT7_HUMAN |
| #54 | CHRNA3 | 80205 | Q3L8U1 | CHD9_HUMAN |
| #55 | RGS17 | 26575 | Q9UGC6 | RGS17_HUMAN |
| #56 | DENND1B | 153090 | Q5VWQ8 | DAB2P_HUMAN |
| #57 | FOXO3 | 56776 | Q9NZ56 | FMN2_HUMAN |
| #58 | RNF112 | 7732 | Q9ULX5 | RN112_HUMAN |
| #59 | ATP6V1G2 | 534 | O95670 | VATG2_HUMAN |
| #60 | ARHGEF10L | 55160 | Q9HCE6 | ARGAL_HUMAN |
| #61 | HRK | 8739 | 000198 | HRK_HUMAN |
| #62 | RIMS3 | 9783 | Q9UJD0 | RIMS3_HUMAN |
| #63 | APBA2 | 321 | Q99767 | APBA2_HUMAN |
| #64 | DIRAS2 | 1612 | P53355 | DAPK1_HUMAN |
| #65 | CAMK2G | 818 | Q13555 | KCC2G_HUMAN |
| #66 | REEP1 | 54463 | Q9H6L5 | RETR1_HUMAN |
| #67 | PRKCE | 5581 | Q02156 | KPCE_HUMAN |
| #68 | SPEG | 10290 | Q15772 | SPEG_HUMAN |
| #69 | KCNQ5 | 56479 | Q9NR82 | KCNQ5_HUMAN |
| #70 | CELF4 | 10659 | 095319 | CELF2_HUMAN |
| #71 | SPOCK1 | 6695 | Q08629 | TICN1_HUMAN |
| #72 | LRRTM2 | 4133 | P11137 | MTAP2_HUMAN |
| #73 | AKAP6 | 9472 | Q13023 | AKAP6_HUMAN |
| #74 | CADPS | 8618 | Q9ULU8 | CAPS1_HUMAN |
| #75 | ATP8A1 | 10396 | Q9Y2Q0 | AT8A1_HUMAN |
| #76 | SLC4A8 | 9498 | Q2Y0W8 | S4A8_HUMAN |
| #77 | FYN | 2487 | Q92765 | SFRP3_HUMAN |
| #78 | SYT1 | 6857 | P21579 | SYT1_HUMAN |
| #79 | SVOP | 55530 | Q8N4V2 | SVOP_HUMAN |
| #80 | CTNND2 | 57482 | Q6ZU35 | CRACD_HUMAN |
| #81 | PRKACB | 5567 | P22694 | KAPCB_HUMAN |
| #82 | PKD1 | 5310 | P98161 | PKD1_HUMAN |
| #83 | CAP2 | 10486 | P40123 | CAP2_HUMAN |
| #84 | BICD1 | 636 | Q96G01 | BICD1_HUMAN |
| #85 | TMOD2 | 29767 | Q9NZR1 | TMOD2_HUMAN |
| #86 | CBFA2T2 | 352954 | Q8NAP1 | CAST3_HUMAN |
| #87 | GAB2 | 2534 | P06241 | FYN_HUMAN |
| #88 | MYT1L | 23148 | 015069 | NACAD_HUMAN |
| #89 | GNB5 | 10681 | 014775 | GNB5_HUMAN |
| #90 | SPTAN1 | 6709 | Q13813 | SPTN1_HUMAN |
| #91 | NRSN1 | 140767 | Q8IZ57 | NRSN1_HUMAN |
| #92 | ARVCF | 421 | 000192 | ARVC_HUMAN |
| #93 | PLCXD2 | 257068 | Q0VAA5 | PLCX2_HUMAN |
| #94 | HMP19 | 51617 | Q6IAL2 | Q61AL2_HUMAN |
| #95 | CDKL2 | 961 | Q08722 | CD47_HUMAN |
| #96 | SRRM4 | 84530 | A7MD48 | SRRM4_HUMAN |
| #97 | YPEL1 | 29799 | O60688 | YPEL1_HUMAN |
| #98 | GATS | 2624 | P23769 | GATA2_HUMAN |
| #99 | GRHL1 | 29841 | Q9NZI5 | GRHL1_HUMAN |
| #100 | CNKSR2 | 22866 | Q8WXI2 | CNKR2_HUMAN |
| #101 | KIFAP3 | 22920 | Q92845 | KIFA3_HUMAN |
| #102 | B3GAT1 | 27087 | Q9P2W7 | B3GA1_HUMAN |
| #103 | SCG5 | 6447 | P05408 | 7B2_HUMAN |
| #104 | SNAP25 | 6616 | P60880 | SNP25_HUMAN |
| #105 | SOX4 | 6659 | Q06945 | SOX4_HUMAN |
| #106 | DPYSL3 | 1809 | Q14195 | DPYL3_HUMAN |
| #107 | MAPT | 4137 | P10636 | TAU_HUMAN |
| #108 | ARSD | 414 | P51689 | ARSD_HUMAN |
| #109 | SULT4A1 | 25830 | Q9BR01 | ST4A1_HUMAN |
| #110 | KCNB1 | 3745 | Q14721 | KCNB1_HUMAN |
| #111 | SNAP91 | 9892 | 060641 | AP180_HUMAN |
| #112 | MYT1 | 23040 | Q9UL68 | MYT1L_HUMAN |
| #113 | CCDC136 | 153733 | Q8NEF3 | CC112_HUMAN |
| #114 | STXBP5L | 9515 | Q9Y2K9 | STB5L_HUMAN |
| #115 | DAPK1 | 1501 | Q9UQB3 | CTND2_HUMAN |
| #116 | RALGPS1 | 5937 | P29558 | RBMS1_HUMAN |
| #117 | MCF2L | 10962 | Q13015 | AF1Q_HUMAN |
| #118 | RIMBP2 | 23504 | 015034 | RIMB2_HUMAN |
| #119 | SCRG1 | 11341 | 075711 | SCRG1_HUMAN |
| #120 | CCDC88A | 79780 | Q8N4S0 | CCD82_HUMAN |
| #121 | ZNF142 | 7701 | P52746 | ZN142_HUMAN |
| #122 | ELOVL4 | 78986 | Q9BV47 | DUS26_HUMAN |
| #123 | RUFY2 | 55680 | Q8WXA3 | RUFY2_HUMAN |
| #124 | PAFAH1B1 | 5048 | P43034 | LIS1_HUMAN |
| #125 | KIAA1211 | 57498 | Q9ULH0 | KDIS_HUMAN |
| #126 | TMEM130 | 222865 | Q8N3G9 | TM130_HUMAN |
| #127 | KIF5C | 22920 | Q92845 | KIFA3_HUMAN |
| #128 | FAM184A | 165215 | Q6P995 | F171B_HUMAN |
| #129 | SAMD14 | 201191 | Q8IZD0 | SAM14_HUMAN |
| #130 | NACAD | 259232 | Q8IZF0 | NALCN_HUMAN |
| #131 | NCAM1 | 135112 | Q8NI08 | NCOA7_HUMAN |
| #132 | KATNAL1 | 84056 | Q9BW62 | KATL1_HUMAN |
| #133 | GPR153 | 387509 | Q6NV75 | GP153_HUMAN |
| #134 | CLIP3 | 23122 | 075122 | CLAP2_HUMAN |
| #135 | CELF2 | 8999 | Q92772 | CDKL2_HUMAN |
| #136 | MAPK8IP2 | 4137 | P10636 | TAU_HUMAN |
| #137 | DISP2 | 163486 | Q6P3S1 | DEN1B_HUMAN |
| #138 | MYO9A | 4661 | Q01538 | MYT1_HUMAN |
| #139 | TMEM169 | 92691 | Q96HH4 | TM169_HUMAN |
| #140 | FOXP1 | 2309 | O43524 | FOXO3_HUMAN |
| #141 | SNX16 | 64089 | P57768 | SNX16_HUMAN |
| #142 | IGSF3 | 3321 | O75054 | IGSF3_HUMAN |
| #143 | STXBP1 | 6812 | P61764 | STXB1_HUMAN |
| #144 | STX1A | 6804 | Q16623 | STX1A_HUMAN |
| #145 | GNG2 | 54331 | P59768 | GBG2_HUMAN |
| #146 | NRXN2 | 9379 | Q9P2S2 | NRX2A_HUMAN |
| #147 | FAM163A | 57579 | Q9P2D6 | F135A_HUMAN |
| #148 | CLASP2 | 1136 | P32297 | ACHA3_HUMAN |
| #149 | DUSP26 | 1804 | P42658 | DPP6_HUMAN |
| #150 | PPP1R12A | 4659 | 014974 | MYPT1_HUMAN |
| #151 | MAP2 | 4216 | Q9Y6R4 | M3K4_HUMAN |
| #152 | GPR137C | 283554 | Q8N3F9 | G137C_HUMAN |
| #153 | BRSK2 | 89927 | Q96MC5 | MERB1_HUMAN |
| #154 | KIF3C | 3798 | Q12840 | KIF5A_HUMAN |
| #155 | ACVR2A | 92 | P27037 | AVR2A_HUMAN |
| #156 | MAST1 | 23263 | 015068 | MCF2L_HUMAN |
| #157 | ANKRD12 | 23253 | Q6UB98 | ANR12_HUMAN |
| #158 | TIA1 | 7072 | P31483 | TIA1_HUMAN |
| #159 | STMN2 | 11075 | Q93045 | STMN2_HUMAN |
| #160 | TULP4 | 56995 | Q9NRJ4 | TULP4_HUMAN |
| #161 | TUBB2A | 7280 | Q13885 | TBB2A_HUMAN |
| #162 | CACNA1B | 774 | Q00975 | CAC1B_HUMAN |
| #163 | GLRB | 2743 | P48167 | GLRB_HUMAN |
| #164 | KLF7 | 54800 | Q6TFL4 | KLH24_HUMAN |
| #165 | SCRN1 | 9805 | Q12765 | SCRN1_HUMAN |
| #166 | ZNF25 | 219749 | P17030 | ZNF25_HUMAN |
| #167 | RUSC1 | 23623 | Q9BVN2 | RUSC1_HUMAN |
| #168 | PCDHB15 | 56121 | Q9Y5E8 | PCDBF_HUMAN |
| #169 | GKAP1 | 80318 | Q5VSY0 | GKAP1_HUMAN |
| #170 | RSPRY1 | 89970 | Q96DX4 | RSPRY_HUMAN |
| #171 | SPAG9 | 9043 | 060271 | JIP4_HUMAN |
| #172 | PI4KA | 5297 | P42356 | PI4KA_HUMAN |
| #173 | FMN2 | 56975 | Q8IXL6 | FA20C_HUMAN |
| #174 | VANGL2 | 57216 | Q9ULK5 | VANG2_HUMAN |
| #175 | ZSWIM6 | 57688 | Q9HCJ5 | ZSWM6_HUMAN |
| #176 | AP3B2 | 8120 | Q13367 | AP3B2_HUMAN |
| #177 | STARD9 | 57519 | Q9P2P6 | STAR9_HUMAN |
| #178 | MYCBP2 | 4644 | Q9Y4I1 | MYO5A_HUMAN |
| #179 | HAND2 | 9464 | P61296 | HAND2_HUMAN |
| #180 | RAB11FIP2 | 5884 | O75943 | RAD17_HUMAN |
| #181 | KLHL24 | 10314 | 043813 | LANC1_HUMAN |
| #182 | APC2 | 10297 | O95996 | APCL_HUMAN |
| #183 | DAB2IP | 1,028E+12 | P49674 | KC1E_HUMAN |
| #184 | CACNB3 | 784 | P54284 | CACB3_HUMAN |
| #185 | KIDINS220 | 3797 | 014782 | KIF3C_HUMAN |
| #186 | CD47 | 55704 | Q3V6T2 | GRDN_HUMAN |
| #187 | C16orf45 | 9024 | Q8IWQ3 | BRSK2_HUMAN |
| #188 | GTF2IRD1 | 9569 | Q9UHL9 | GT2D1_HUMAN |
| #189 | SP4 | 6671 | Q02446 | SP4_HUMAN |
| #190 | CCDC82 | 64753 | Q96JN2 | CC136_HUMAN |
| #191 | PREPL | 9581 | Q4J6C6 | PPCEL_HUMAN |
| #192 | EHBP1 | 1809 | Q14195 | DPYL3_HUMAN |
| #193 | AKT3 | 10000 | Q9Y243 | AKT3_HUMAN |
| #194 | OGG1 | 4968 | 015527 | OGG1_HUMAN |
| #195 | CCDC112 | 9139 | O43439 | MTG8R_HUMAN |
| #196 | ARID1B | 57492 | Q8NFD5 | ARI1B_HUMAN |
| #197 | MARCKS | 4139 | Q9P0L2 | MARK1_HUMAN |
| #198 | PCMT1 | 5110 | P22061 | PIMT_HUMAN |
| #199 | DMXL2 | 85455 | A7MBM2 | DISP2_HUMAN |
| #200 | RBMS1 | 65055 | Q9H902 | REEP1_HUMAN |
| #201 | ENO2 | 23301 | Q8NDI1 | EHBP1_HUMAN |
| #202 | FAM20C | 79632 | Q8NB25 | F184A_HUMAN |
| #203 | AUTS2 | 26053 | Q8WXX7 | AUTS2_HUMAN |
| #204 | CHD9 | 56853 | Q9BZC1 | CELF4_HUMAN |
| #205 | CNTFR | 22866 | Q8WXI2 | CNKR2_HUMAN |
| #206 | RPAIN | 84268 | Q86UA6 | RIP_HUMAN |
| #207 | WDR47 | 22911 | O94967 | WDR47_HUMAN |
| #208 | PGAP1 | 80055 | Q75T13 | PGAP1_HUMAN |
| #209 | SLC9A6 | 10479 | Q92581 | SL9A6_HUMAN |
| #210 | TSPYL4 | 23270 | Q9UJ04 | TSYL4_HUMAN |
| #211 | PCMTD2 | 55251 | Q9NV79 | PCMD2_HUMAN |
| #212 | DOPEY2 | 1740 | Q15700 | DLG2_HUMAN |
| #213 | HACE1 | 57531 | Q8IYU2 | HACE1_HUMAN |
| #214 | MAP4K4 | 23542 | Q13387 | JIP2_HUMAN |
| #215 | TCF4 | 6925 | P15884 | ITF2_HUMAN |
| #216 | IRF2BP2 | 359948 | Q7Z5L9 | I2BP2_HUMAN |
| #217 | SENP7 | 57337 | Q9BQF6 | SENP7_HUMAN |
| #218 | ZNF33A | 7581 | Q06730 | ZN33A_HUMAN |
| #219 | HERC1 | 8925 | Q15751 | HERC1_HUMAN |
| #220 | EPC2 | 6785 | Q9GZR5 | ELOV4_HUMAN |
| #221 | PHIP | 55023 | Q8WWQ0 | PHIP_HUMAN |
| #222 | SMARCC2 | 6601 | Q8TAQ2 | SMRC2_HUMAN |
| #223 | SCAMP5 | 192683 | Q8TAC9 | SCAM5_HUMAN |
| #224 | LANCL2 | 1902 | Q92633 | LPAR1_HUMAN |
| #225 | FAM135A | 2045 | Q15375 | EPHA7_HUMAN |
| #226 | PLEKHA1 | 59338 | Q9HB21 | PKHA1_HUMAN |
| #227 | MAP3K4 | 9448 | 095819 | M4K4_HUMAN |
| #228 | ARGLU1 | 55082 | Q9NWB6 | ARGL1_HUMAN |
| #229 | AGAP1 | 116987 | Q9UPQ3 | AGAP1_HUMAN |
| #230 | RUFY3 | 22902 | Q7L099 | RUFY3_HUMAN |
| #231 | FAM171B | 148753 | Q96GL9 | F163A_HUMAN |
| #232 | ZEB1 | 6935 | P37275 | ZEB1_HUMAN |
| #233 | CSNK1E | 7464 | Q92828 | COR2A_HUMAN |
| #234 | MARK1 | 22983 | Q9Y2H9 | MAST1_HUMAN |
| #235 | ZMIZ1 | 57178 | Q9ULJ6 | ZMIZ1_HUMAN |
| #236 | SPIN1 | 10927 | Q9Y657 | SPIN1_HUMAN |
| #237 | RAD17 | 9649 | Q5JS13 | RGPS1_HUMAN |
| #238 | LANCL1 | 55915 | Q9NS86 | LANC2_HUMAN |
| #239 | SGTB | 54557 | Q96EQ0 | SGTB_HUMAN |
| #240 | MLLT11 | 23077 | O75592 | MYCB2_HUMAN |
| #241 | POGZ | 23126 | Q7Z3K3 | POGZ_HUMAN |
| #242 | YWHAH | 7533 | Q04917 | 1433F_HUMAN |

In some embodiments of the invention, the gene is selected from the group consisting of: *Transcription factor SOX-6 (SOX6;* GenelD 55553, updated 26.04.21); *Neurexophilin-1 (NXPH1;* GenelD 30010, updated 26.04.21); *Peripherin (PRPH; GenelD 5630,* updated *26.04.21); Transcription Factor AP-2 Beta (TFAP2B;* GenelD 7021, updated 26.04.21*); Ephrin type-A receptor 7 (EPHA7; GenelD 2045,* updated 26.04.21*); Sodium channel protein type 2 subunit alpha (SCN2A; GenelD 6326,* updated 26.04.21); *V-set and transmembrane domain-containing protein 2A (VSTM2A, Gene ID 222008,* updated 26.04.21*); Sodium channel protein type 9 subunit alpha (SCN9A; Gene ID 6335,* updated 26.04.21*); Plexin-A4 (PLXNA4; Gene ID 91584,* updated 26.04.21); *Sodium channel protein type 3 subunit alpha (SCN3A; Gene ID 6328,* updated 26.04.21*); Talin-2 (TLN2; Gene ID 83660,* updated 26.04.21); *Contactin-associated protein-like 2 (CNTNAP2; Gene ID 26047,* updated 26.04.21*); Adenylate cyclase type 1 (ADCY1; Gene ID 107,* updated 26.04.21*); RGM domain family member B (RGMB, Gene ID 285704,* updated 26.04.21*); PH and SEC7 domain-containing protein 2 (PSD2; Gene ID 84249,* updated 26.04.21).

The term "core SA signature" as used herein refers to the transcriptional signature shared by at least 3 of the 4 cell sympatho-adrenal cell types involved the development of medulla of the human adrenal gland.

The cluster c1 contains the 338 genes that are i) expressed at higher levels in both LR-NB and HR-NB samples compared to samples of the healthy fetal adrenal gland, and ii) expressed at higher levels in LR-NBs than in HR-NBs.

The SA-c1 module is a sub-cluster of the cluster c1, comprising 242 genes characterized as relevant in NB formation. This sub-cluster comprises transcription factors (TFs) SOX6 and TFAP2, involved in the early stages of the NCC lineage; PRPH and the TFs GATA2 and FOXO3, which are associated with chromaffin cell differentiation; and various autism spectrum disorder genes *(CNTNAP2, CTNND2, DLG2, NRXN2* and *SHANK2)* recently associated with NB aetiology.

In the present invention, the terms polypeptide(s) and protein(s) are used interchangeably. The term "protein(s) of interest" is understood as those proteins encoded by the corresponding genes of interest. The term "gene(s) of interest" is understood as any gene selected from the SA-c1 module (Table 1).

One of the genes comprising the SA-c1 module is *NXPH1.* The protein NXPH1 encoded by the gene is also known as NPH1 (UniProt ID P58417 V131, last modified 12.08.20).

The α-NRXN-specific ligands (NXPHs) comprise a family of four glycoproteins (NXPH1-4). In contrast to other α-NRXN ligands expressed throughout the brain in virtually all excitatory and inhibitory neurons, the NXPH family is restricted to certain inhibitory neuronal subpopulations. While the function of NXPHs is poorly understood, data suggest that they block the interaction of α-NRXNs with post-synaptic proteins within the neural system.

In higher mammals, NXPH1-3 bind α-NRXN1. NXPH1-3 are small 29 kDa, secreted, neuropeptide-like proteins found in a variety of tissues. NXPHs are preferentially expressed in the brain but they are also present in non-neural tissues in a species-specific pattern. In the mouse, NXPH1-3 are expressed primarily in small subsets of inhibitory neurons of the nervous system. In humans, the strongest expression of NXPH1 is detected in the adrenal gland.

NXPH1-3 are highly evolutionarily conserved in mammals. Whilst NXPH1 is the best-characterized member of the family, NXPH2 and NXPH3 are expected to undergo a similar maturation process due to their high degree of sequence conservation and to present comparable, even possibly overlapping, functions. Each gene codes for a variable N-terminal region and a highly conserved C-terminal region. In neural tissues, the immature form of NXPH1 undergoes N-glycosylation at its C-terminal domain and a proteolytic cleavage of the N-terminal domain, thus giving rise to a mature neuropeptide-like form that binds to the extracellular domain of the transmembrane receptors α-NRXNs. NXPH1 plays a role in synaptogenesis and synapse maturation by regulating the accessibility of other ligands to the extracellular domain of α-NRXNs. To date, there is no evidence that NXPH1 plays other functions and the intracellular signaling consequences of its binding to α-NRXNs remain unknown. However, it is worth noting that a loss of *NXPH1* expression correlates with the malignant progression of both pancreatic ductal adenocarcinoma and breast cancer. NXPH1 has also been proposed as a DNA methylation biomarker associated with a good prognosis for NB patients.

In mammals, the highly conserved NRXN family consists of three genes: neurexin 1, neurexin 2 and neurexin 3. Each *NRXN* gene possesses two independent promoters driving the expression of 2 different isoforms: alpha (α)- and beta (β)-NRXNs. All the NRXN isoforms are type-1 transmembrane proteins containing an isoform-specific extracellular domain followed by a single transmembrane region and a short cytoplasmic PDZ domain. The β-NRXNs represent an N-terminal truncated form of α-NRXNs and present a specific short N-terminal sequence. Thus, most α-NRXN ligands (including NXPHs) are not able to bind to β-NRXNs. NRXNs are widely expressed in the nervous system, where all three genes (NRXN1-3) are transcribed at similar levels and play key roles in synaptic formation and neurotransmission. NRXNs are located at the synaptic cleft where they constitute trans-synaptic signaling complexes together with different pre- and post-synaptic ligands and transmembrane proteins. Pre-synaptic NRXNs send an instructive signal that induces the initial organization of the post-synaptic bouton. They moreover contribute to synaptic refinement in a neuronal subtype-specific manner. Consistent with their relevant role in synaptic neurotransmission, genetic alterations in the NXRNs genes are associated with various neuropsychiatric and neurodevelopmental disorders.

In some embodiments, the activator of the gene modulates the specification and early development of the neural crest cells and their derivatives. Among the multiple derivatives of this embryonic population, the sympatho-adrenal lineage will give rise to the peripheral nervous system (PNS), which comprises the chromaffin cells of the adrenal medulla, the paravertebral sympathetic ganglia chain and additional prevertebral ganglia such as the celiac ganglia, the suprarenal ganglia and the mesenteric ganglia

The PNS controls many physiological functions including heart rhythm, blood pressure, perspiration and salivation. Alterations in the formation or function of the PNS cause several pediatric disorders, including NB.

The present invention comprises the use of a gene activator (hereinafter "activator of the invention"). In the present invention, the term "gene activator" or variants thereof refer to any molecule capable of enhancing the biological activity of the gene by any mode of action, including but not limited to inducing the expression of the gene and increasing the levels of the biological activity of the polypeptide encoded by the gene (hereinafter "protein of interest").

In some embodiments of the invention, the activator of the invention might, for example, and among others, increase the biological activity of an extracellular polypeptide encoded by the gene i.e., *NXPH1* or *FRZB* by binding to and activating its receptor.

The activators of the invention may be identified using methods based on (i) the analysis of the migratory and invasive capacity of NB tumour cells *in vitro,* including but not restricted to scratch assays, transmembrane assays, microfluidic chamber assays or cell exclusion zone assays; as previously reported (Puls et al., 2018 Scientific Reports 8, 13039.; Evensen et al., 2013 PloS ONE 8(12): e82811.; and Hulkower, K.I., et al., 2011 Pharmaceutics 3(1): 107-124); or (ii) the analysis of the metastatic potential of NB tumour cells *in vivo* using experimental models of metastasis in mouse and/or chick animal models, as reported by the inventors in their manuscript or by others (Delloye-Bourgeois et al., 2017 Cancer Cell 32, 427-443.e8; Palmer et al., 2011 J Vis Exp 51: 2815; Sethi et al., 2011 Cancer Cell 19(2): 192-205 and Zijlstra et al., 2002 Cancer Cell 13(3): 221-234).

Any activator may be used in the use of the present invention. However, in a particular embodiment of the use of the invention, the activator may be selected from the group consisting of: (i) an antibody or fragment thereof; (ii) a small molecule; (iii) an expression activator of the gene of interest; and (iv) an expression activator of a gene encoding a receptor, wherein said receptor binds a polypeptide encoded by *NXPH1* or *FRZB.*

In some embodiments, the activator is an antibody or fragment thereof.

The term "antibody" is understood as a polypeptide including at least a light chain or heavy chain immunoglobulin variable region which specifically recognizes and binds an epitope of a polypeptide, such as a receptor or a fragment thereof. Antibodies are composed of a heavy and a light chain, each of which has a variable region, termed the variable heavy (VH) region and the variable light (VL) region. Together, the VH region and the VL region are responsible for binding the antigen recognized by the antibody. Antibodies of the present disclosure include those that are specific for the receptors of the extracellular protein of interest (NXPH or FRZB) and enhance the biological activity of the protein of interest.

The term "antibody" includes intact immunoglobulins, as well the variants and portions thereof, such as Fab' fragments, F(ab)'2 fragments, single chain Fv proteins ("scFv"), and disulfide stabilized Fv proteins ("dsFv"). A scFv protein is a fusion protein in which a light chain variable region of an immunoglobulin and a heavy chain variable region of an immunoglobulin are bound by a linker, while in dsFvs, the chains have been mutated to introduce a disulfide bond to stabilize the association of the chains. The term also includes genetically engineered forms such as chimeric antibodies (for example, humanized murine antibodies) or heteroconjugate antibodies (such as diabodies or bispecific antibodies).

The antibodies can be prepared using any of the methods which are known by the person skilled in the art, some of which have been mentioned above. Thus, the polyclonal antibodies are prepared by means of immunizing an animal with the protein to be targeted. The monoclonal antibodies are prepared using the method described by Kohler and Milstein et al. 1975 (Nature 256: 495-497). Once antibodies with a specific receptor binding capacity are identified, those capable of enhancing the activity of the extracellular protein of interest will be selected using a relevant identification assay.

The antibody may be an agonist. In the context of the present invention, the term "agonist" is understood as any molecule capable of binding specifically to the same receptors as an extracellular protein of interest and activating one or more functions of said protein. Alternatively, the antibody may bind an inhibitor of a protein of interest. The term "inhibitor" refers to any chemical or biological substance that reduces or suppresses the activity of the protein of interest.

The activator may also be a small molecule. The activator small molecules according to the invention are molecules, typically with a molecular weight less than about 1000 Daltons, or in some embodiments, less than about 500 Daltons, wherein the molecule is capable of modulating, to some measurable extent, the activity of a target molecule, such as a polypeptide encoded by one of the genes of interest in the present invention.

The small molecule may be an agonist, as described above.

The term "(gene) expression activator" or any variants thereof is understood as any activator which increases the levels of the expression of a gene, for example by controlling the transcription or the translation of the gene.

Within the present invention, the expression activator may increase the levels of expression of the genes/proteins of interest or the levels of expression of the gene encoding a receptor binding an extracellular protein of interest. Methods for inducing the expression of the gene encoding the protein include, without being limited to, editing technologies such as CRISPR/cas9 or Cas9 nickase technology.

The present invention relates to a gene activator for use in the treatment and/or prevention of NB malignancies.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above).

As used herein, the term "preventing" (or "prevent" or "prevention") refers to the capacity of the gene activator to minimize or hinder the progression of a disease, condition or disorder in a subject.

In the present invention, the condition or disorder to be treated or prevented is a NB malignancy. As used herein, the term "NB malignancies" is used to refer to any malignancies that develop from certain very early forms of nerve cells and chromaffin cells, also known as immature sympatho-adrenal progenitor cells, found in several areas of the body.

The stage of the disease may be determined according to the International Neuroblastoma Staging System (INSS), developed taking into account the degree of tumour resection, presence of ipsilateral or contralateral lymph nodes involvement, tumour infiltration across the midline of the body, and separation of patients with INSS stage 4S from other children with metastatic disease (INSS stage 4) to harmonize staging across groups. The different stages and their key characteristics are shown in Table 2.

**Table 2. International Neuroblastoma Staging System (INSS).**

| **INSS Stage** | **Description** |
|---|---|
| 1 | Localized tumour, grossly resected, no lymph node involvement |
| 2A | Unilateral tumour, incomplete gross excision, negative lymph nodes |
| 2B | Unilateral tumour, with or without incomplete gross excision, with positive ipsilateral lymph nodes |
| 3 | Unresectable tumour infiltrating across midline or unilateral tumour with contralateral lymph nodes or midline tumour with bilateral lymph nodes |
| 4 | Distant metastatic disease |
| 4S | Localized primary tumour as defined by stage 1 or 2 in patient under 12 months with dissemination limited to the liver, skin, and/or bone marrow (<10% involvement) |

The INRG Staging System, or INRGSS, is a more recent staging system which utilizes image-defined risk factors in place of degree of surgical resection. The different stages and their key characteristics are shown in Table 3.

**Table 3. International Neuroblastoma Risk Group Staging System (INRGSS).**

| **INRG Stage** | **Description** |
|---|---|
| L1 | Localized tumour with no image-defined risk factors |
| L2 | Localized tumour with one or more image-defined risk factors |
| M | Distant metastatic disease |
| MS | Metastatic disease in children under 18 months with metastases limited to skin, liver, and/or bone marrow (<10% involvement) |

In the INRG classification system, a combination of clinical, pathologic, and genetic markers are used to predict the clinical behavior of the tumour and how it will respond to treatment. These markers are used to define risk, and include age at the time of diagnosis, stage of the disease, histologic category, the grade or how cells of the tumour are differentiated, *MYCN* gene status, chromosome 11q status, and tumour cell ploidy.

According to these markers, NB can be classified into one of four categories: very low-risk, low-risk, intermediate-risk, or high-risk. HR-NBs include stage L1 with *MYCN* amplification, stage L2 with *MYCN* amplification, stage M in children younger than 18 months of age with *MYCN* amplification, stage M in children with older than 18 months, stage MS in children younger than 18 months with 11q aberration and stage MS in children younger than 18 months of age with *MYCN* amplification.

In some embodiments of the invention the NB malignancy to be treated is a HR-NB malignancy.

In some embodiments of the invention, the stage of the neuroblastoma malignancy is INSS4. In some embodiments of the invention, the NB malignancy carries amplification of the oncogene *MYCN.*

In particular embodiments of the invention, the activator inhibits the metastatic potential of the NB malignancy. In the context of NB, the term 'metastatic potential' refers to the capacity of the NB cells to colonize and grow in organs or locations other than those forming the peripheral nervous system. This includes, but is not restricted to, the bone marrow, the bones, the lymph nodes and/or the liver.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a preferred embodiment, the subject is a human, man or woman of any age or race.

In some embodiments of the invention, the activator of the invention is comprised within a pharmaceutical composition in a therapeutically effective amount. The term "therapeutically effective amount" means the amount of activator of the invention that will elicit the biological or medical response of a tissue, system, animal or human that is being sought by the researcher, veterinarian, medical doctor or other clinician. In the context of the present invention, the biological or medical response to elicit is the treatment and/or prevention of NB malignancies.

The activators of the present invention can be incorporated into pharmaceutical compositions for its administration to the subject. The activators of the invention may conveniently be presented in dosage unit form and may be prepared by any of the methods well known in the art of pharmacy. All methods include the step of bringing the active ingredient into association with the carrier that constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleagenous suspension. This suspension may be formulated according to the known art using those suitable dispersing or wetting agents and suspending agents that have been mentioned above. The sterile injectable preparation may also be a sterile injectable solution or suspension in a non-toxic parenterally acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

The activator of the invention may also be administered in the form of suppositories for rectal administration of the drug. These compositions can be prepared by mixing the drug with a suitable non-irritating excipient that is solid at ordinary temperatures but liquid at the rectal temperature and will therefore melt in the rectum to release the drug. Such materials are cocoa butter and polyethylene glycols.

In the treatment, prevention, control, amelioration, or reduction of risk of NB malignancies, an appropriate dosage level of the activator of the invention will generally be about 0.01 to 500 mg per kg patient body weight per day, which can be administered in single or multiple doses. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1,000.0 milligrams of the activator for the symptomatic adjustment of the dosage to the patient to be treated.

It will be understood, however, that the specific dose level and frequency of dosage for any particular patient may vary and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The activator of the present invention may be used in combination with one or more other drugs in the treatment, prevention, control, amelioration, or reduction of risk of NB malignancies, where the combination of the drugs together are safer or more effective than either drug alone. Thus, in particular embodiments of the use of the invention, the pharmaceutical composition further comprises a compound for the treatment of NB malignancies in a subject.

Examples of compounds useful for treating NB malignancies which may be used in conjunction with the activator of the invention include, without being limited to, cisplatin, vincristine, carboplatin, etoposide, cyclophosphamide, dinutuximab and isotretinoin.

The activators of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual or buccal routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the activators of the invention are effective for use in humans.

Such other compound(s)/drug(s) may be administered by a route and in an amount commonly used therefore, simultaneously or sequentially with the activator of the invention. When the activator is used simultaneously with one or more other drugs, a pharmaceutical composition in unit dosage form containing such other drugs and the activator is preferred. However, the combination therapy may also include therapies in which the activator of the invention and one or more other drugs are administered on different overlapping schedules. When used in combination with one or more other active ingredients, the activator of the invention and the other active ingredients may be used in lower doses than when each is used singly. Accordingly, the pharmaceutical compositions of the present invention include those that contain one or more other active ingredients, in addition to an activator.

In some embodiments, the activator of the invention is an activator of the *NXPH1* gene. In some particular embodiments, the activator of the invention is an activator of the *α-NRXN1* gene.

In another aspect, the present invention relates to an *in vitro* method for diagnosing NB malignancies in a subject, comprising: (i) determining the expression levels of at least one gene selected from the group consisting of the genes listed in Table 1 in a biological sample from the subject, and (ii) comparing the expression levels obtained in step (i) with a first reference expression level obtained in a fetal adrenal gland sample from a subject not suffering from NB malignancies, wherein if the expression levels of the at least one gene listed in (i) are increased with respect to the first reference expression level, then the subject is suffering from an NB malignancy.

In some embodiments, the *in vitro* method for diagnosing NB malignancies in a subject comprises determining the expression levels of at least one gene selected from the group consisting of: *SOX6; NXPH1; PRPH; TFAP2B; EPHA7; SCN2A; VSTM2A; SCN9A; PLXNA4; SCN3A; TLN2; CNTNAP2; ADCY1; RGMB;* and *PSD2* in a biological sample from the subject.

Whilst increased expression levels of *NXPH1; PRPH; TFAP2B; EPHA7; SCN2A; VSTM2A; SCN9A; PLXNA4; SCN3A; TLN2; CNTNAP2; ADCY1; RGMB;* and *PSD2* may be associated to both LR-NB and HR-NB malignancies in a subject, increased expression levels of *SOX6* are generally associated to LR-NB malignancies, and decreased expression levels of *SOX6* are generally associated to HR-NB malignancies.

Therefore, in some specific embodiments, the *in vitro* method for diagnosing NB malignancies in a subject comprises:
(i) determining the expression levels of at least one gene selected from the group consisting of: *NXPH1; PRPH; TFAP2B; EPHA7; SCN2A; VSTM2A; SCN9A; PLXNA4; SCN3A; TLN2; CNTNAP2; ADCY1; RGMB;* and *PSD2* in a biological sample from the subject; and/or
(ii) determining the expression levels of *SOX6* in a biological sample from the subject; and
(iii) comparing the expression levels obtained in step (i) with a first reference expression level obtained in a fetal adrenal gland sample from a subject not suffering from NB malignancies,
wherein if the expression levels of the at least one gene listed in (i) are increased with respect to the first reference expression level, and/or the expression levels of SOX6 are increased or decreased with respect to the first reference expression level, then the subject is suffering from an NB malignancy.

As used herein, an expression level increased with respect to a reference expression level refers to an expression level that is at least: 5% higher, 8% higher, 10% higher, 15% higher, 20% higher, 25% higher, 30% higher, 35% higher, 40% higher, 45% higher, 50% higher, 55% higher, 60% higher, 65% higher, 70% higher, 75% higher, 80% higher, 85% higher, 90% higher, 95% higher, 96% higher, 97% higher, 98% higher, 99% higher, 100% higher, 1-fold higher, 2-fold higher, 5-fold higher, 10 fold higher, 20 fold higher, 30 fold higher, 40 fold higher, 50 fold higher, 60 fold higher, 70 fold higher, 80 fold higher, 90 fold higher, 100 fold higher, 1000-fold higher, or more, than the first reference expression level.

In some embodiments, the expression levels of at least one gene selected from the group consisting of: *NXPH1; PRPH; TFAP2B; EPHA7; SCN2A; VSTM2A; SCN9A; PLXNA4; SCN3A; TLN2; CNTNAP2; ADCY1; RGMB; PSD2;* and/or *SOX6* are determined in the biological sample from the subject.

In some embodiments of the invention, the expression levels of two or more genes selected from the group consisting of: *NXPH1; PRPH; TFAP2B; EPHA7; SCN2A; VSTM2A; SCN9A; PLXNA4; SCN3A; TLN2; CNTNAP2; ADCY1; RGMB; PSD2;* and/or *SOX6* are determined in the biological sample from the subject.

In some specific embodiments of the invention, the expression levels of *NXPH1; PRPH; TFAP2B; EPHA7; SCN2A; VSTM2A; SCN9A; PLXNA4; SCN3A; TLN2; CNTNAP2; ADCY1; RGMB; PSD2;* and/or *SOX6* are determined in the biological sample from the subject.

In some embodiments, the expression levels of *NXPH1; PRPH; TFAP2B; EPHA7; SCN2A; VSTM2A; SCN9A; PLXNA4; SCN3A; TLN2; CNTNAP2; ADCY1; RGMB;* or *PSD2* in the sample are at least 4-fold higher than the first reference expression level.

In some embodiments, the expression levels of *NXPH1; PRPH; TFAP2B; EPHA7; SCN2A; VSTM2A; SCN9A; PLXNA4; SCN3A; TLN2; CNTNAP2; ADCY1; RGMB;* or *PSD2* in the sample are at least 10-fold higher than the first reference expression level.

In some embodiments, the expression levels of *SOX6* in the sample are at least 3-fold higher than the first reference expression level.

The term "an expression level decreased with respect to the first reference expression level" refers to an expression level that is at least: 5% lower, 8% lower, 10% lower, 15% lower, 20% lower, 25% lower, 30% lower, 35% lower, 40% lower, 45% lower, 50% lower, 55% lower, 60% lower, 65% lower, 70% lower, 75% lower, 80% lower, 85% lower, 90% lower, 95% lower, 96% lower, 97% lower, 98% lower, 99% lower, 100% lower, 1-fold lower, 2-fold lower, 5-fold lower, 10 fold lower, 20 fold lower, 30 fold lower, 40 fold lower, 50 fold lower, 60 fold lower, 70 fold lower, 80 fold lower, 90 fold lower, 100 fold lower, 1000-fold lower, or more, than the second reference expression level.

In some specific embodiments, the expression levels of *SOX6* in the sample are at least 3-fold lower than the first reference expression level.

In some embodiments of the invention, the *in vitro* method may further comprise: (iii) comparing the expression levels obtained in step (i) with a second reference expression level obtained in a biological sample from a subject diagnosed with a LR-NB malignancy; and (iv) comparing the expression levels obtained in step (i) with a third reference expression level obtained in a biological sample from a subject diagnosed with a HR-NB malignancy, wherein (a) if the expression levels of the genes listed in (i) are decreased with respect to the second reference expression level and comparable to the third reference expression level, then the subject is susceptible to receiving treatment based on an activator of the invention.

As used herein, an expression level decreased with respect to the second reference expression level refers to an expression level that is at least: 5% lower, 8% lower, 10% lower, 15% lower, 20% lower, 25% lower, 30% lower, 35% lower, 40% lower, 45% lower, 50% lower, 55% lower , 60% lower, 65% lower, 70% lower, 75% lower, 80% lower, 85% lower, 90% lower, 95% lower, 96% lower, 97% lower, 98% lower, 99% lower, 100% lower, 1-fold lower, 2-fold lower, 5-fold lower, 10 fold lower, 20 fold lower, 30 fold lower, 40 fold lower, 50 fold lower, 60 fold lower, 70 fold lower, 80 fold lower, 90 fold lower, 100 fold lower, 1000-fold lower, or more, than the second reference expression level.

In some embodiments, the expression levels of *NXPH1; PRPH; TFAP2B; EPHA7; SCN2A; VSTM2A; SCN9A; PLXNA4; SCN3A; TLN2; CNTNAP2; ADCY1; RGMB;* or *PSD2* in the sample are at least 2.5-fold lower than the second reference expression level.

In some embodiments, the expression levels of *NXPH1; PRPH; TFAP2B; EPHA7; SCN2A; VSTM2A; SCN9A; PLXNA4; SCN3A; TLN2; CNTNAP2; ADCY1; RGMB* or *PSD2* in the sample are at least 5-fold lower than the second reference expression level.

In some embodiments, the expression levels of *SOX6* in the sample are at least 1-fold lower than the second reference expression level.

In some embodiments, the expression levels of *SOX6* in the sample are at least 2-fold lower than the second reference expression level.

An expression level comparable to a reference expression level is any expression level which is not increased or decreased with respect the reference level, as defined herein.

All the relevant terms have been explained above in the present description, and these explanations, as well as its particular embodiments, are applicable to the present inventive aspect.

Methods for determining the expression levels of a gene are widely known in the state of the art, and any of them can be used in the context of the present invention. The determination of the expression levels of the genes of interest may comprise measuring the level of cDNA, the level of mRNA, and/or the level of the protein encoded by said gene. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the gene of interest (gene encoding the protein) or of the corresponding cDNA thereof, S1 nuclease mapping, hybridisation, RT-Q-PCR, micro-array and RNA-sequencing, etc. If the quantification of the expression of the gene encoding the protein is going to be carried out from the protein of interest, then the isolated biological sample of the subject must be treated to extract the protein of interest. Methods for extracting or isolating proteins are known by the person skilled in the art and they are commercially available. The levels of gene encoding the protein of interest can be quantified by means of any conventional method that enables said protein to be detected and quantified in a sample of a subject. By way of illustration and not limitation, the levels of said protein can be quantified, for example, by means of the use of antibodies with the capacity to bind specifically to the protein of interest and the subsequent quantification of the complexes formed.

Accordingly, in some embodiments of the invention, the decreased or increased expression as mentioned above may lead to a decrease or increase in the transcription rate of a gene of about: 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the reference sample.

Similarly, in some embodiments, the decreased or increased expression may lead to a decrease or increase in the amount of mRNA of a gene of about 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the reference sample.

In some embodiments of the invention, the decreased or increased expression may lead to a decrease or increase in the amount of the polypeptide encoded by the gene of about 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the reference sample.

The term "reference level" refers to the expression levels of the gene encoding the protein of interest in a sample of a subject other than the subject being diagnosed with the method of the invention. Specifically, the term "first reference level" refers to the expression levels of the gene encoding the protein of interest in a fetal adrenal gland sample from a subject not suffering from NB malignancies ("control individual/s"). The term "second reference level" refers to the expression levels of the gene encoding the protein of interest in a sample of a subject diagnosed with a LR-NB malignancy. The term "third reference level" refers to the expression levels of the gene encoding the protein of interest in a sample of a subject diagnosed with a HR-NB malignancy.

The levels of expression may be determined using any type of biological sample from the subject. As used herein, the term "biological sample" refers to any material comprising a nucleic acid and/or a polypeptide of interest. Examples of biological samples include, but without limiting to, a biopsy sample, a tissue (e.g., brain tissue), cell or fluid (e.g., serum, saliva, semen, sputum, cerebral spinal fluid (CSF), tears, mucus, sweat, brain extracts and the like) and blood (e.g., PBMCs (peripheral blood-derived mononuclear cells) such as neutrophils, monocytes).

In some embodiments of the invention, the levels of expression of the gene of interest are determined in a blood sample from the subject. In preferred embodiments of the invention, the levels of expression of *NXPH1* and *FRZB* are determined in a blood sample from the subject.

As described above, if the expression levels of the at least one gene listed in (i) are decreased with respect to the second reference expression level and comparable with respect to the third reference expression level, then the subject is susceptible to receive a therapy based on an activator of the invention.

Thus, in another preferred embodiment of the method of the invention, the activator is selected from the group consisting of (i) an antibody or fragment thereof; (ii) a small molecule; (iii) an activator of the gene expression; and (iv) an expression activator of a gene encoding a receptor, wherein the receptor binds a polypeptide encoded by *NXPH1* or *FRZB.*

All these activators have been defined and explained for the use of the invention, and, as understood by the skilled person, all of them are applicable to the present method of the invention.

In certain embodiments of the invention, the subject is a human subject.

In certain embodiments of the invention, the NB malignancy of the *in vitro* method is a HR-NB malignancy, as defined above.

In certain embodiments of the invention, the gene of interest is *NXPH1.*

In further inventive aspects, the present invention also encompasses a method of treating or preventing NB malignancies in a subject, comprising enhancing the activity of the gene of interest, as described herein, and the use of a gene activator as described herein in the manufacture of a medicament for the treatment of NB malignancies in a subject. All the preferred embodiments and explanations of the terms used in these inventive aspects have been explained above.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Figure 1****.** NB formation is correlated with a neural signature accentuated in LR-NBs.
**Figure 2****.** The core sympatho-adrenal signature is enriched in LR-NBs and associates with better patient prognosis.
**Figure 3****.** The expression of NXPH1 and its receptors α-NRXN1/2 associates with favorable patient prognosis and identifies NB cells with a neural crest stem cell identity.
**Figure 4****.** The activity of NXPH1 and α-NRXN1 is required for NB growth.
**Figure 5****.** The inhibition of NXPH1 or α-NRXN1 activity unleashes the metastatic potential of NB cells.
**Figure 6****.** Genome-wide transcriptomic analysis of neuroblastoma formation and malignancy and event-free survival probability of the top 3 candidate genes of clusters c1-c4.
**Figure 7****.** Gene set enrichment analyses of distinct signatures related to human adrenal gland development.
**Figure 8****.** Comparison of the transcript levels of the top 15 SA-c1 genes in fAG, LR-NB and HR-NB samples.
**Figure 9****.** Event-free survival probabilities of NB patient samples based on the expression levels of SA-c1 genes, *NXPH1* or *NRXN1-3.*
**Figure 10****.** Cellular behaviour of constitutive and inducible sh-NXPH1 and sh-αNRXN1 clones in vitro.
**Figure 11****.** The addition of rNXPH1 stimulates growth and increases the proportion of P75/NTR+ NB cells in the CAM assay.

### EXAMPLES

### Materials and Methods

**Animal Studies.** Fertilized white Leghorn chicken eggs were provided by Granja Gibert, rambla Regueral, S/N, 43850 Cambrils, Spain. Eggs were incubated in a humidified atmosphere at 38ºC in a Javier Masalles 240N incubator, manipulated at embryonic day 10 (E10) and sacrificed at E17. Sex was not identified.

5 weeks-old female NOD/SCID mice (NOD.CB17-Prkdcscid /NCrHsd; RRID: IMSR_ARC: NODSCID) and 7 weeks-old male NSG mice (NOD.Cg-Prkdcscidll2rgtm1Wjl/SzJ; RRID: BCBC_4142) provided by Envigo were used for subcutaneous xenografting and metastasis analyses, respectively. Mice were housed under a regimen of 12 hours light/12 hours dark cycles in specific pathogen-free conditions. Sterile dry pellets and water was administered ad libitum. Animals were sacrificed by cervical dislocation or CO2 administration at the end of the experiment or when required for ethical reasons.

**Patient-derived xenografts.** Human NB-PDX models (HSJD-NB-012, HSJD-NB-011 and HSJD-NB-007) were established, maintained and provided by A.M.C under a local animal care and use committee-approved protocol (135/11). All NB-PDX models were derived from patients with progressive stage INSS4 disease, refractory to all treatments. HSJD-NB-011 was established from a 2.5-year-old male, with amplification of MYCN gene, TP53 wild type and ALK-mutated (I1171N). HSJD-NB-007 was established from a 5-year-old male with amplification of MYCN gene and no mutations in TP53 and ALK. HSJD-NB-012 was established from a 4-year-old male and its genetic profile is unknown. HSJD-NB-011 and HSJD-NB-007 have been previously used for publication (Monterrubio et al, J Control Release. 2017;255:108-19; Boeva et al, Nat Genet. 2017;49(9):1408-13). Experimental manipulation of NB-PDXs was performed in HSJD under the supervision of A.M.C.

To quantify α-NRXN1 expression in PDXs, tumours were dissected off the flank of immunocompromised nude mice, minced using razor blades in a sterile dish and transferred to a 50ml falcon tube. Cells were dissociated following manufacturer instructions using the Brain Tumour Dissociation Kit and a gentle MACS dissociator (Milteny Biotec #130-095-942 and #130-093-235). Red blood cells were removed by using ACK buffer. A single cell suspension weas prepared in FACS buffer (ice-cold DMEM-5%FBS) containing 10µM of Rock inhibitor (SIGMA #Y0503) to a final concentration of 1·107 cells/ml and stained for flow cytometry analysis as explained below

**Cell lines.** HEK-293 (RRID: CVCL_0045), LAN-1 (RRID: CVCL_1827), IMR-5 (RRID: CVCL_1306), SH-SY5Y (RRID: CVCL_0019), SK-N-AS (RRID: CVCL_1700), SK-N-JD (RRID: CVCL_WH12), SK-N-LP (RRID: CVCL_WH13), LA1-5s (RRID: CVCL_2549), SK-N-SH (RRID: CVCL_0531), SL-N-Be(2)c (RRID: CVCL_0529) and IMR-32 (RRID: CVCL_0346) cell lines were obtained from Marian Martínez-Balbás (IBMB-CSIC), Cinzia Lavarino (HSJD) and Joan Xavier Comella (VHIR). NB cell lines were grown in either RPMI 1640-Glutamax (ThermoFisher Scientific cat# 61870010) supplemented with 10% (LAN-1, IMR-5, SH-SY5Y, SK-N-AS, SK-N-JD, SK-N-LP, LA1-5s) or 20% (SK-N-SH, SK-N-Be(2)c, IMR-32) foetal bovine serum (FBS; Cultek cat# 16SV30160.03RYB35908) plus 1% penicillin/streptomycin antibiotic cocktail (ThermoFisher Scientific cat# 15140-122). The HEK-293 cell line was grown in DMEM-Glutamax (Lifetechnologies cat#61965-026) supplemented 10% FBS and 1% penicillin/streptomycin cocktail. Unless indicated, cells were grown in standard culture conditions. The presence of mycoplasma contamination was routinely checked by immunofluorescence. Cell lines have not been re-authenticated for the present paper.

**Genome-wide transcriptomic analysis.** The HSJD-NB dataset used in this study has been published previously (Gomez S et al, Epigenomics. 2015;7(7):1137-53) and is available at GEO repository (GSE54720). The full human SCP, bridge cell, chromaffin cell and sympathoblast signatures were obtained from a published study (Kildisiute et al, Sci Adv. 2021 Feb 5;7(6):eabd3311), and the complementary signatures were obtained from a Venn diagram analysis performed with a freely available software developed by the group of Dr Y. Van de Peer (VIB, Brussels, Belgium, http://bioinformatics.psb.ugent.be/webtools/Venn/).

Genome-wide transcriptomic analyses were performed using the web-based Phantasus software (https://artyomovlab.wustl.edu/phantasus; version 1.5.1) (Zenkova et al, 2018; Available from: https://genome.ifmo.ru/phantasus). Normalized log expression values were obtained using the Quantile Normalize Adjustment tool. The Maximun Median Probe method was then selected to collapse the different probes of a single gene. Finally, genes were filtered based on raw expression levels and the 12,000 most-expressed genes from each array were selected for subsequent analysis. Sample dispersion was then assessed using the Principal Component Analysis method and detected outliers were removed from further analysis. Differentially-expressed genes (DEGs) between groups were identified using the Limma R package. The genes were considered as differentially expressed when the false discovery rate (FDR)-adjusted P-value (with Benjamini-Hochberg procedure) was <0.05. Gene set enrichment analyses (GSEA) were performed using the FGSEA tool from Phantasus.

**Gene ontology analysis.** Gene ontology (GO) term enrichment analyses (biological process) were performed with the PANTHER classification system (http://pantherdb.org) (Mi et al, Nucleic Acids Res. 2019;47(D1):D419-D426; Su et al, Genome Biol. 2014;15((12)):523). The 50 most-enriched GO terms were used to assess GO term distribution and were annotated with the Ancestor Chart tool of QuickGO (https://www.ebi.ac.uk/QuickGO).

R2 genomic analysis and visualization platform. Publically available NB patient microarray SEQC 498 (GSE62564) was obtained from the R2 genome analysis and visualization platform (https://hgserver1.amc.nl/cgi-bin/r2/main.cgi). The R2-web based application was used to generate Kaplan-Meier event-free survival curves. Data were grouped based on the expression levels of either NXPH1, NRXN1-3 or the full SA-c1 gene module. In the latter case, the SEQC cohort was subdivided into quartiles based on the combined expression of the 242 SA-c1 genes, considering as "high" or "low" the expression of each SA-c1 gene with respect to its own average expression level. The quartiles Q1-Q4 thereby consisted of 123-126 samples showing numbers of SA-c1 genes expressed at high levels as follows: 487≤Q1≤360; 359≤Q2≤250; 249≤Q3≤135 and 134≤Q4≤4. Statistical significance was automatically assessed by the R2-server using a log-rank test. The R2-web based application was also used to compare the distribution of patients among different NB prognosis groups according to expression levels of the SA-c1 gene module or NXPH1. For the analysis of disease progression, the category "progression" included tumors that did not respond to therapy plus those that were recurrent despite an initial therapeutic response. Statistical significance was automatically assessed by R2-server using the Chi-square + Fisher's test.

**Plasmids and lentiviral infection.** Inhibition of NXPH1 and α-NRXN1 expression was triggered by lentiviral infection of short-hairpin constructs inserted into in pLKO.1 or doxycycline-inducible pSLIK-Neo vectors (Moffat et al, Cell. 2006;124(6):1283-98; Shin et al, Proc Natl Acad Sci U S A. 2006;103(37):13759-64). The pLKO.1-TRC cloning vector (RRID: Addgene_10878) and the pLKO.1-sh-Ctl (RRID: Addgene_10879) were kindly provided by Marian Martínez-Balbás. Doxycycline-inducible miRshRNA-eGFP-expressing pSLIK-Neo lentiviral vectors were generated by gateway recombination between the TTRE-eGFP-miR-shRNA entry vector and the pSLIK destination vector (Shin et al, Proc Natl Acad Sci U S A. 2006;103(37):13759-64). pEN_TTGmiRc2 and pSLIK-Neo were purchased from Addgene (Cat#25753, RRID: Addgene_25752 and Cat#25735, RRID: Addgene_25735; gifts from Ian Fraser). NXPH1 and α-NRXN1-specific sh-RNA sequences were designed using the web-based Genetic Perturbation Platform of the Broad Institute (https://portals.broadinstitute.org/gpp/public/). Expression of luciferase was triggered by lentiviral infection of pLEX-hFL2iG vector.

Lentiviral particles were generated using the psPAX2 (RRID: Addgene_12260) and pMD2.G plasmids (RRID: Addgene_12259) kindly provided by Marian Martínez-Balbás (IBMB-CSIC). Briefly, HEK293T cell line was transfected using the Lipofectamine 2000 Tranfection Reagent (ThermoFisher Scientific #11668019) following manufacturer's instruction and lentiviruses were harvested 48h post-transfection. Transduction of target cells was usually performed readily after lentivirus recovery. 2µg/ml of puromycin (SIGMA #p8833) and 600µg/ml of neomycin/G418 (SIGMA-ALDRICH #A1720) were used for selection of transduced cells.

RNA purification and RT-qPCR. Total RNA was extracted with TRIzol Reagent (ThermoFisher Scientific #15596-018) and Pellet Paint Co-precipitant (Merck Millipore #69049-3). Genomic DNA traces were removed with DNA-free DNase Treatment and Removal Reagents (FisherScientific #AM1906) following manufacturer's instructions. DNA-free RNA was retro-transcribed using the High-Capacity cDNA Reverse Transcription Kit (LifeTechnlogies #4368814). Semi-quantitative PCR was performed using the Lightcycler 480 SYBR Green 2x Master (Roche #04887352001) on a Roche Lightcycler 480 Real-Time PCR System. Unless indicated otherwise, primers were design using the UCSC Genome browser and Primer3Plus online softwares. 18SrRNA or TBP were used as housekeeping genes. Relative cDNA levels were calculated using the efficiency-corrected ΔCt method (Bookout et al, Curr Protoc Mol Biol. 2006;15:15.8). Heatmap representations were performed using GraphPad Prism v6 (RRID: SCR_002798).

**Fluorescence-activated cell sorting (FACS).** All experiments were conducted using a BD FACSAria Fusion at the Cytometry Core Facility (Centres Científics i Tecnològics) of Universitat de Barcelona. Briefly, single cell suspensions coming from in vitro cell cultures or dissociated PDXs were prepared in FACS buffer (ice-cold DMEM-5%FBS) at 107 cells/ml and incubated for 60min at 4°C with the following fluorescent-conjugated antibodies diluted in FACS buffer: rat monoclonal anti-CD31 (clone 390), PE-Cy^{™}7 (BD Pharmingen Cat#561410, RRID: AB_10612003), rat monoclonal anti-CD11b (clone M1/70), PE-Cy^{™}7 (BD PharMingen Cat#561098, RRID: AB_2033994), mouse monoclonal anti-Disialoganglioside GD2 (clone 14.G2a), Alexa Fluor^{®} 647 (BD PharMingen Cat#562096, RRID: AB_1154051) and a rabbit polyclonal anti-Neurexin 1α, ATTO488 (Alomone Labs Cat#ANR-031-AG, RRID: AB_2756687). Finally, cells were washed twice with ice-cold FACS washing buffer and resuspended at 5·106 cells/ml in ice-cold FACS buffer. Dox-induced cells from sh-Ctrl, sh-NXPH1 and sh-αNRXN1 clones were purified based on eGFP sorting. DAPI (0.1µg/ml) or propidium iodide (2µg/ml) were used as vital dyes

The gating strategy for detection and purification of α-NRXN1 subpopulations was adapted from a previous study (Merlos-Suarez et al, Cell Stem Cell. 2011;8(5):511-24). The 10-12% of NB cells showing the strongest mean fluorescent intensity were used as α-NRXN1 + and the 20-40% with the lowest intensity were used as α-NRXN1. Among α-NRXN1+ cells, the 0.5-1% of cells showing the highest intensity were considered as α-NRXN1 High and the 10-20% with an intensity 3 to 4 times lower as α-NRXN1Low. For depletion experiments, the 10-15% of cells showing the brightest NRXN1-A488 intensity was discarded. FACS data was analyzed with FlowJo (RRID: SCR_000410).

**MTT assay.** Approximately 8,000 cells were seeded in quadruplicates. At desired timepoints, 10µl of 5mg/ml MTT solution (SIGMA #M2128) was added to each well and incubated at 37°C for 4 hours. Formazan crystals were dissolved with 100µl DMSO (SIGMA #D2650) and the optical density at 570nm (OD570) was measured. This assay was performed on n=2-4 biological replicates.

**S-phase index and BrdU-retention assay.** A solution containing 10 µM of 5-bromo-2'-deoxyuridine (BrdU; SIGMA #858811) was added to the growth medium of SK-N-SH cell monolayers and cells were incubated at 37ºC for 2 hours to estimate the S-phase index, or for 24 hours prior to the CAM assay to assess BrdU retention, using n=4-9 biological replicates. Cells were then fixed, permebilized and incubated with 5U/ml DNase I type II (SIGMA #D4527) for 15 min at RT. BrdU incorporation was then assessed by immunofluorescence as described below.

**Tumour spheroid assay.** NB cells were grown in sphere-forming media (SFM) containing DMEM/F12 (ThermoFisher Scientific #11330-032), 10% KnockOut serum replacement (ThermoFisher Scientific #10828012), 2mM Glutamax (ThermoFisher Scientific #35050038), 1% MEM non-essential amino acids (ThermoFisher Scientific #11140035), 0.1mM β-mercaptoethanol (SIGMA-ALDRICH #M3148), 1% penicillin/streptomycin cocktail (ThermoFisher Scientific #15140-122) and 0.1ng/ml basic FGF (ThermoFisher Scientific #PHG0021). Medium was renewed every week. The sphere-forming capacity of the different human NB cell lines was performed on n=2 biological replicates.

To assess mRNA expression in stem cell-enrichment conditions, 3·106 cells were grown on uncoated 60mm plates in 2ml SFM. Spheres were allowed to grow for 5 weeks and were photographed using an inverted bright-field microscope (Leica DMIRBE). Viable spheres were recovered by centrifugation at 100g for 5min at 4ºC) and processed for RNA extraction as described below.

To assess the sphere-forming capacity of NB cells, 2,000 dissociated cells from each population of interest were sorted in quadruplicates and seeded directly onto 12-well plates coated with 0.5% agarose (Condisa Laboratory #8014). Cells were allowed to grow for 1 week and the presence of spheroids was monitored every two days. To detect the presence of viable spheroids after 1 week, plates were incubated with 0.5mg/ml MTT (SIGMA #M2128) for 3 hours at 37°C. Viable spheres (black coloured) were manually counted using an inverted bright-field microscope (Leica DMIRBE). The sphere-forming capacity was assessed for n=1-4 biological replicates per experimental condition.

**2D Extreme limiting dilution assay (ELDA).** Cell subpopulations were FACS-sorted, seeded in triplicates into 96-well plates and grown until one experimental condition fully covered the well surface. Half of the growth medium was renewed every week. Cells were then fixed with 4% PFA (SIGMA #16005) for 15min at RT, washed with PBS and stained with 0.5% crystal violet (SIGMA #V5365) for 20 min at RT. The dye excess was washed out and plates were air-dried for at least 2 hours at RT. Crystal violet staining was dissolved by adding 200µl methanol to each well, followed by 20min at RT. Finally, the OD570 was measured. This assay was performed on n=3 biological replicates per experimental condition.

**Immunofluorescence.** Fixed samples (either cells growing in vitro or tumor sections) were permeabilized with 0.1% Triton X100 in PBS (0.1% PBT) for 15 minutes at RT, saturated for 30 min in a blocking buffer (10% horse serum, 1% BSA, 0,3% Glycine in 0.1% PBT), and incubated overnight at 4°C with primary antibodies diluted in blocking buffer. After 3 washes in PBS, samples were incubated with fluorescence-conjugated secondary antibodies for 2 hours at RT. Samples were stained with rat monoclonal anti-BrdU [clone BU1/75 (ICR1)] (BioRad Cat #MCA 2060, RRID: AB_323427), rabbit monoclonal anti-active Caspase-3 (clone C92-605) (BD PharMingen Cat#559596, RRID: AB_397274), rabbit polyclonal anti-phospho-Histone H3 (Ser10) (Upstate Cat#06-570, RRID: AB_310177), rat monoclonal anti-phospho-Histone H3 (pSer28) (clone HTA28) (Sigma Cat#H9908, RRID: AB_260096), mouse monoclonal anti-Human Nuclear Antigen (clone 235-1) (Abcam Cat#ab191181, RRID: AB_2492189), rabbit polyclonal anti-Ki67 (Abcam Cat#ab66155; RRID: AB:1140752), mouse monoclonal anti-p75NTR (clone ME20.4) (Millipore Cat#05-446, RRID: AB_309737) and mouse monoclonal anti-PSA-NCAM (clone 2-2B) (AbCys Cat#AbC0019, RRID: AB_2313692). Cell nuclei were stained with 1 µg/ml DAPI and samples mounted in home-made Mowiol. At least n=4 independent samples were assessed for each experimental condition.

Image acquisition and treatment. Optical sections of human NB cell samples were acquired at RT with the Leica LAS_X (2016) software on an Automated Inverted Leica AF7000 wide-field microscope using 20x (Dry/NA 0.5/HC PL APO/0.70 CS∞/0.17/C) or 40x (oil/NA 1.25-0.75/HCX PL APO Lbd Blue ∞/0.17/D). Cell counting was performed in minimum of 4 fields per well using the ImageJ software (https://imagej.nih.gov/ij/; RRID: SCR_003070) (Schindelin et al, Nature Methods. 2012. p. 9(7):676-82; Rueden et al, BMC Bioinformatics. 2017;18(1):529).

Optical sections of NB tumour graft samples were acquired at RT with the Leica LAS software (RRID: SCR_013673), in a Leica TCS SP5 confocal microscope using 20x (dry/HC PL APO 20x/0.70 CS ∞/0.17/C) or 40x (oil /HCX PL APO 40X/1.25-0.75 OIL CS ∞/0.17/D) objective lenses or with the LSM Software ZEN 2.1 (RRID: SCR_018163), in a ZEISS Lsm780 confocal microscope using 25x (oil,w,Glic, NA 0.8, Plan-Apochromat/ImmKorr) or 40x (oil/NA 1.3/Plan-Apochromat/(UV)VIS-IR). Whole sections of NB tumour grafts were acquired as tile scans using the motorized XY stage. Tiles were directly stitched into a single mosaic by the microscope software. Maximal projections obtained from 3-6µm Z-stack images were processed with ImageJ for image merging, resizing and cell counting. Cell counting was performed in a minimum of two sections per tumour.

**Cell counting.** Two Fiji macros were developed for cell counting. The first one uses the DAPI channel to create a binary mask containing all the nuclei; segmentation is based on local contrast enhancing, filtering and a manual pipeline for fine tuning. The second one uses the so-produced binary mask to extract the nuclei selections and collate them against the original images, in order to intensity threshold the nuclei for the three markers used: GFP, Ki67 and pH3. The macro delivers a result table and a processed compound image with all the nuclei labelled according to their content. Codes and the details on the procedure can be downloaded at https://github.com/MolecularlmagingPlatformlBMB/CellProliferationAssay.

**Tumour Xenografts.** SK-N-SH clones carrying a conditional knockdown of NXPH1 (sh-NXPH1), α-NRXN1 (sh-αNRXN1) or a scramble control sequence (sh-Ctrl) were first activated in vitro in presence of 1mg/ml doxycycline (ACEFESA #PAA29510025) for 96 hours. Pre-activated cells were then sorted by FACS based on eGFP expression, and resuspended in grafting media consisting of DMEM/F12, 7mg/ml Matrigel (Fisher Scientific #10365602, 1mg/ml doxycycline and 10µM of Rock inhibitor (SIGMA #Y0503) at a concentration of 0.5·107 cells/ml. Then, 100µl cell suspensions were grafted subcutaneously using a 0.5ml BD Micro-Fine 29G insulin syringe. Each animal received 2 grafts, one on each flank. A fresh solution containing 2mg/ml of doxycycline dissolved in 7.5% sucrose-bearing sterile water was administered ad libitum in drinking water, its administration starting 2 days prior to xenografting. Mouse weight and tumour growth were monitored twice a week until tumour detection. From this point, the tumour volume was measured every two days with a digital calliper and mice were sacrificed once the tumour volume reached ethical permission limits. Tumours were then resected, weighted and photographed.

**Experimental metastasis assay.** SK-N-SH clones carrying a luciferase cassette and a conditional knockdown of NXPH1 (sh-NXPH1), α-NRXN1 (sh-αNRXN1) or a scramble control sequence (sh-Ctrl) were first activated in vitro in presence of 1mg/ml doxycycline for 96 hours. To test the metastatic potential of NB cells, 2·105 cells resuspended in 100µl of PBS were injected into the left cardiac ventricle of 7 weeks-old male NSG mice (NOD.Cg-Prkdcscid ll2rgtm1Wjl/SzJ) with a 26G ½ 13mm needle, using a total of 7 injected mice per experimental condition. 2mg/ml of doxycycline dissolved in 7.5% sucrose-bearing sterile water was administered ad libitum in drinking water as explained before. The generation of metastases was monitored twice per week by bioluminescence imaging (BLI) with the IVIS Imaging System (Perkin Elmer) after retro-orbital administration of 15µg/µl D-Luciferin (GoldBio #LUCK-10G). Metastatic lesions were quantified by measuring the photon count of the region of interest, using an exposure time of 2 minutes and a medium binning value. Images taken at time "2 min" post-injection are presented with a higher threshold (10^7) than images taken at later time points to show the even cell distribution throughout the body right after injection.

**Chick CAM assay.** At E10, a small window was created on the egg shell using a sterile scalpel. Cells resuspended in grafting media consisting of DMEM/F12, 7mg/ml Matrigel and 10µM of Rock inhibitor were implanted onto the surface of the chorio-allantoid membrane (CAM) by gently scraping the upper CAM layer (avoiding bleeding or visible rupture of the capillaries). To assess the effects of NXPH1 on tumour growth, parental SK-N-SH cells were trypsinized and resuspended in grafting media in presence of 10µg/ml recombinant NXPH1 (rNXPH1) or 100µg/ml BSA (Ctrl; SIGMA #A7906). To test the growth of NB cells depleted from their α-NRXN1+ subpopulation, parental SK-N-SH depleted or not from their α-NRXN1+ subpopulation were FACS-purified and resuspended in grafting media. In both experiments, a concentration of 0.5·106 cells/10µl was used and grafted onto the CAM, using n=4-13 biological replicates. At E17 NB tumour grafts were removed from the CAM and tumours were photographed, weighted and measured using a digital calliper (VWR). The final tumour volume (V) was calculated using the following formula: V= 4/3·π·length·depth·width. Tumours were then fixed in 4% PFA for 1h30min at 4°C, washed in PBS1x and sequentially cryo-protected with PBS solutions containing 15% and 30% sucrose. Small cubical tumour pieces (5mm·5mm·5 mm approx.) were then embedded in Tissue-tek (Sakura Finetek #4583) and frozen on dry ice. Tumour pieces were sectioned on a cryostat (Leica) at 16µm, collected serially on home-made TESPA pre-coated slides and slides stored at -20°C. Immunofluorescence was performed as described above.

**Statistics.** No statistical method was used to predetermine sample size. The experiments were not randomized. The investigators were not blinded to allocation during experiments. Statistical analyses were performed using the GraphPad Prism 6 software (RRID: SCR_002798). Unless noted otherwise (see quantifications), cell counts were typically performed on 3 images per sample and n values correspond to different tumors or biological replicates. The normal distribution of the values was assessed by the Shapiro-Wilk normality test. Significance was then assessed with a two-way ANOVA + Dunnett's or Tukey's test for data presenting a normal distribution, or alternatively with the non-parametric Mann-Whitney test, the Mantel-Cox log-rank test or the Kruskal-Wallis test + Dunn's test for non-normally distributed data. For survival analysis, significance was assessed with the Gehan-Breslow-Wilcoxon test. Statistical analysis performed on the GSE62564 dataset was automatically assessed by the R2 server (https://hgserver1.amc.nl/cgi-bin/r2/main.cgi) using the Mantel-Cox log-rank test or the Chi-square + Fisher's test. The following convention was used: n.s: P>0.05; *P<0.05, **P<0.01, ***P<0.001. Statistical details of experiments can be found in the Fig. legends and main text above.

**Study approval.** All the experimental procedures involving mice were carried out in accordance with the European Union guidelines (Directive 2010/63/EU) and according to the guidelines from the Animal Care Committee at the Generalitat de Catalunya. The studies were approved by the ethics committee of the Pare Científic de Barcelona and by the Pare de Recerca Biomèdica de Barcelona (PRBB) animal facility policy. According to Spanish animal care guidelines, no approval was required to perform the experiments involving chicken embryos herein. Human NB-PDX models (HSJD-NB-012, HSJD-NB-011 and HSJD-NB-007) were established, maintained and provided by A.M.C under a local animal care and use committee-approved protocol (135/11).

### Results

### Example 1. NB formation is correlated with a neural signature accentuated in LR-NBs.

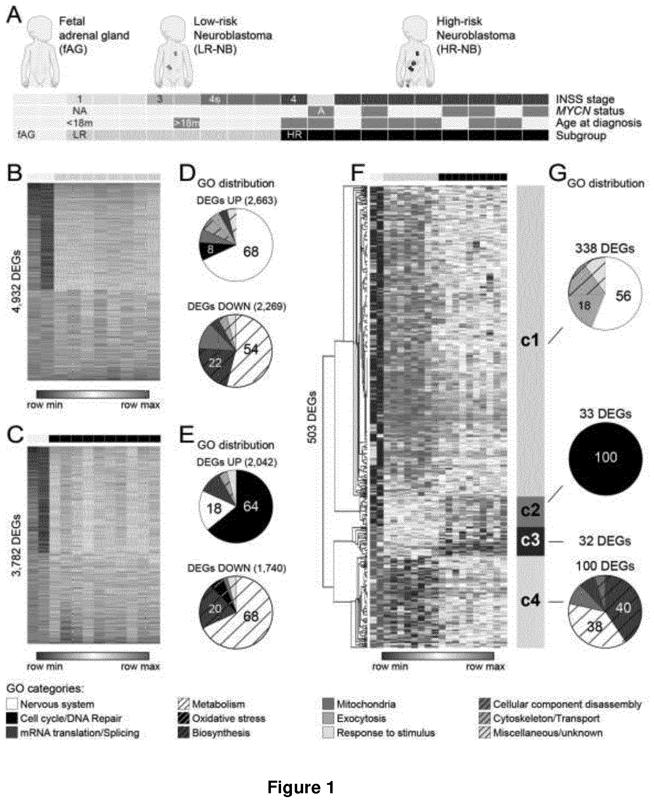

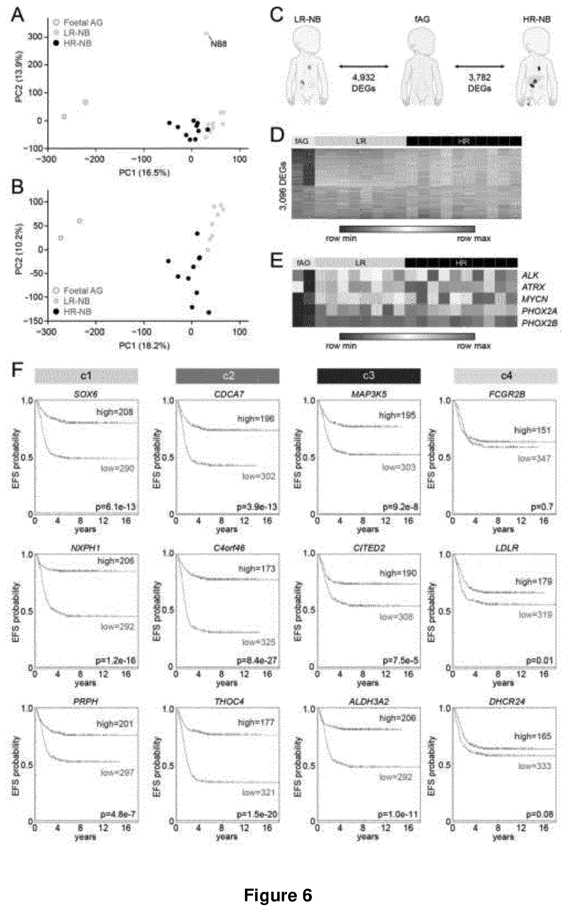

We set out to define the transcriptional signatures associated to the aetiology of LR- and HR-NBs and to the malignant behavior of HR-NBs. As such, we analyzed the transcriptome of primary tumor samples from 19 NB patients. These samples were classified as LR (n=9) or HR (n=10) based on their tumor stage (International Neuroblastoma Staging System INSS 1-3, 4s and 4) (Brodeur et al, J Clin Oncol. 1993;11(8):1466-77), their *MYCN* amplification status and their age at diagnosis (Fig. 1A) (Gomez S et al, Epigenomics. 2015;7(7):1137-53). Samples of the human fetal adrenal gland (fAG) were used as a normal reference tissue (Fig. 1A). A principal component analysis (PCA) confirmed the overall homogeneity of these groups, identifying one outlier sample in the LR-NB group that was removed from subsequent analyses (Fig. 6A and B).

To identify the transcriptional signatures associated to NB aetiology, we first independently compared the LR- and HR-NB groups to the fAG samples and thereby identified 4,932 and 3,782 differentially expressed genes (DEGs), respectively (Fig. 1B, C). A gene ontology (GO) enrichment analysis revealed that the downregulated DEGs retrieved from the LR vs fAG and HR *vs* fAG comparisons were associated mainly with GO terms related to metabolism and biosynthesis (Fig. 1D, E), in agreement with previous findings (De Preter et al, Genome Biol. 7 (2006) doi:10.1186/gb-2006-7-9-r84.). By contrast, the DEGs found upregulated in these two comparisons were both characterized by a marked enrichment in GO terms related to the nervous system, or to the cell cycle and DNA repair (Fig. 1D, E). The GO terms related to the nervous system represented 68% of the top 50 GOs for the LR *vs* fAG comparison but only 18% for the HR *vs* fAG comparison (Fig. 1D, E), indicating that the formation of both LR- and HR-NBs is associated to a neural signature, and that this signature is more pronounced in LR-NBs.

We next searched for a transcriptional signature relevant to NB formation that furthermore distinguishes LR-NBs from HR-NBs. To this aim, we first crossed the two independent comparisons LR *vs* fAG and HR *vs* fAG and thereby retrieved 3,096 common DEGs, which included the well-established markers of NB malignancy: *ALK, ATRX, MYCN, PHOX2A* and *PHOX2B* (Fig. 6C-E). We then searched among these 3,096 common DEGs for the genes that were further differentially expressed between the LR-NB and HR-NB groups. This comparison identified a list of 503 LR *vs* HR DEGs that an unbiased hierarchical clustering subdivided into 4 clusters of genes presenting distinct expression profiles (Fig. 1F). The clusters c1 and c2 consisted of genes more strongly expressed in both LR-NBs and HR-NBs than in fAG samples. The best represented cluster (c1) contained 338 genes that were more strongly expressed in LR-NBs than in HR-NBs and were associated with a marked enrichment in GO terms related to the nervous system (Fig. 1F, G). The expression levels of the top 30 genes found in cluster c1 (such as *SOX6, NXPH1, PRPH*) were all correlated with a favorable patient prognosis, as shown by Kaplan-Meyer analyses done with an independent cohort of 498 NB patients (Fig. 6F). Conversely, the 33 genes found in cluster c2 (such as *CDCA7, C4orf46, THOC4)* were more strongly expressed in HR-NBs than in LR-NBs and presented the features of oncogenes, as shown by their GO term enrichment profile and Kaplan-Meyer analyses (Fig. 1F, G, Fig. 6F). On the other hand, the clusters c3 and c4 consisted of genes expressed more weakly in both LR-NBs and HR-NBs than in fAG samples. The 32 genes found in cluster c3 presented the features of tumor suppressors, while the cluster c4 contained 100 genes that were more expressed in HR-NBs than in LR-NBs and were mainly correlated with a bad prognosis (Fig. 1F, G, Fig. 6F). Our analysis thus identified a complex transcriptional signature relevant to NB formation that furthermore distinguishes LR-NBs from HR-NBs. Remarkably, 67% of the 503 genes found in this signature, which formed the cluster c1, are both associated with a neural identity and a better patient outcome.

### Example 2. The core sympatho-adrenal signature is enriched in LR-NBs and associates with better patient prognosis.

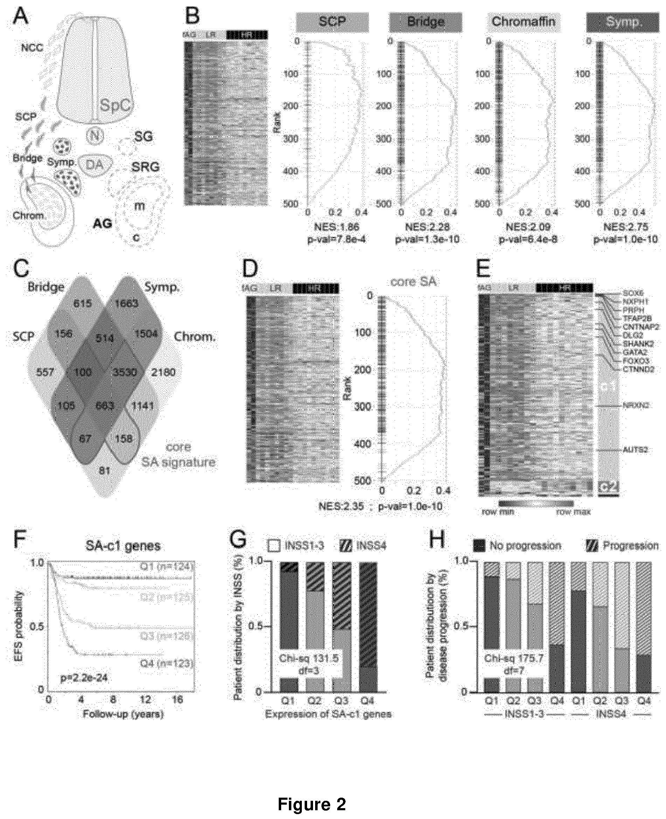

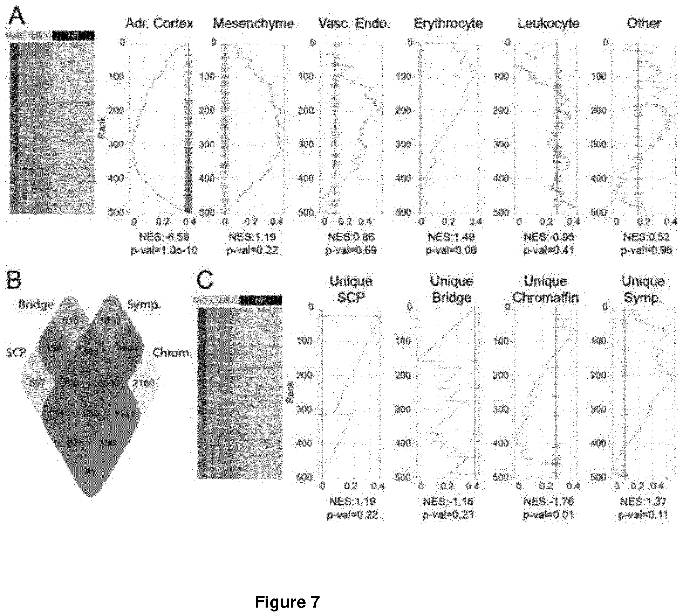

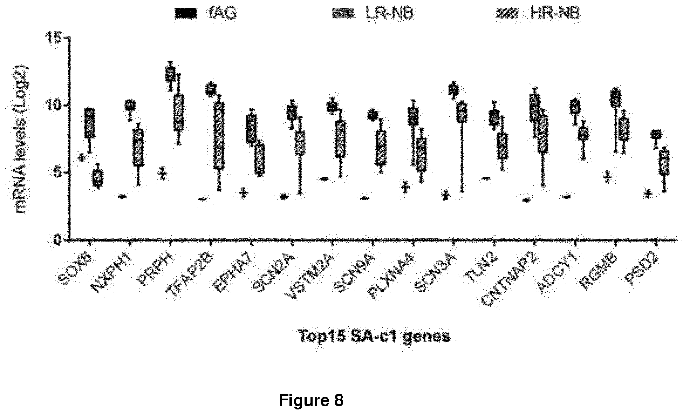

We next assessed whether the transcriptional signature distinguishing LR-NBs and HR-NBs was related to their sympatho-adrenal (SA) origin. Both the mouse and human SA lineages can be subdivided into 4 cell identities: Schwann cell precurors (SCPs), bridge cells, chromaffin cells and sympathoblasts (Fig. 2A) [Furlan et al, Science (80-). 2017;357(6346):eaal3753 ; Dong et al, Cancer Cell. 2020;38(5):716-733.e6; Kildisiute et al, Sci Adv. 2021 Feb 5;7(6):eabd3311; Hanemaaijer et al, Proc Natl Acad Sci U S A. 2021;118(5)]. We searched for their possible enrichment within our list of LR *vs* HR DEGs using transcriptional signatures recently identified for each of these 4 cell identities by single-cell RNA-seq during human adrenal gland development (Kildisiute et al, Sci Adv. 2021 Feb 5;7(6):eabd3311). We thereby observed that the 4 SA cell signatures all showed a strong enrichment for genes expressed at higher levels in LR-NBs than in HR-NBs, retrieving as many as 342 of our 503 DEGs (Fig. 2B). By contrast, the signatures of the non-adrenal medulla cell types identified in the developing human adrenal gland did not show any enrichment, except the adrenal cortex signature which was enriched in genes expressed at higher levels in HR-NBs (Fig. 7A). We noticed that the full SCP, bridge cell, chromaffin cell and sympathoblast signatures were largely overlapping (Fig. 2C). We retrieved a strong enrichment for genes expressed at higher levels in LR-NBs when we used a core SA signature consisting of the 4518 genes shared by at least 3 of the 4 SA cell signatures (Fig. 2D). By contrast, this enrichment was lost when using the signatures specifically consisting of the genes unique to SCPs, bridge cells or sympathoblasts, and the "unique" chromaffin cell signature showed a converse and weak enrichment towards genes expressed at higher levels in HR-NBs (Fig. 7B and C). Using the core SA signature, we retrieved more than half of the 503 LR *vs* HR DEGs (Fig. 2D and Table S6). Even more strikingly, 92% of this list corresponded to genes belonging to the cluster c1 (242 genes out of 262; Fig. 2E, Tables 1 and S6), with the top 15 SA-c1 genes *(SOX6; NXPH1; PRPH; TFAP2B; EPHA7; SCN2A; VSTM2A; SCN9A; PLXNA4; SCN3A; TLN2; CNTNAP2;ADCY1; RGMB; PSD2)* presenting mRNA levels increased by at least 4 folds between LR-NB and HR-NB samples (Fig.8 and Table 1). The transcriptional landscapes of LR-NB and HR-NBs can thus be discriminated based on a signature corresponding to the core SA lineage, and this developmental program is unexpectedly associated with a favorable prognosis.

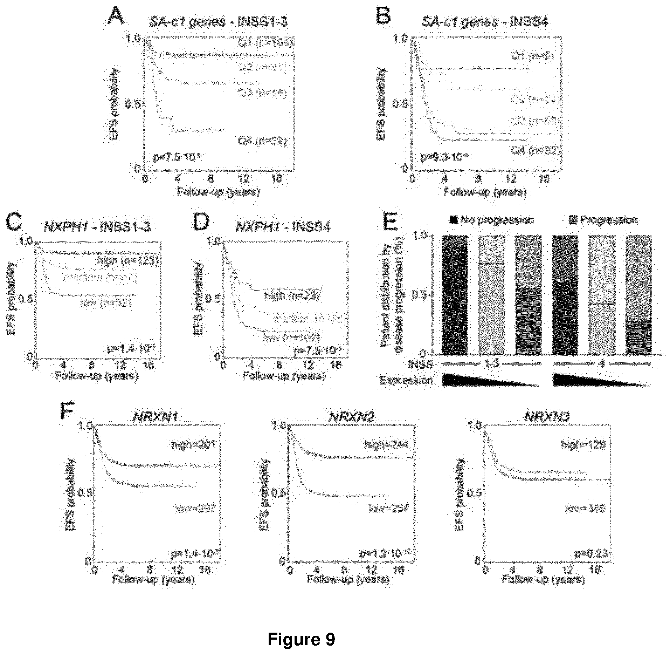

Among these 242 genes common to the core SA signature and to the LR *vs* HR DEGs of the cluster c1 (thereafter named "SA-c1" genes) were found for instance the transcription factors (TFs) SOX6 and TFAP2 involved in the early stages of the NCC lineage (Dong et al, Cancer Cell. 2020;38(5):716-733.e6; Rothstein et al, Genome Res. 2020;30(1):35-48), PRPH and the TFs GATA2 and FOXO3, which are associated with chromaffin cell differentiation (Dong et al, Cancer Cell. 2020;38(5):716-733.e6), and various autism spectrum disorder genes *(CNTNAP2, CTNND2, DLG2, NRXN2* and *SHANK2)* recently associated with NB aetiology (Lopez et al, Genome Res. 2020 Sep;30(9):1228-42) (Fig. 2E, Tables 1). To test the predictive potential of the SA-c1 gene module on NB prognosis, we subdivided the SEQC NB cohort into quartiles based on the combined expression of the 242 SA-c1 genes, whereby the quartiles Q1 and Q4 consisted of the patient samples presenting the highest and lowest numbers of SA-c1 genes expressed above average levels, respectively (see Materials and Methods). These quartiles showed a progressive decrease in event-free survival probability (8-years EFS probability of 0.88, 0.80, 0.49 and 0.27 for Q1, Q2, Q3 and Q4, respectively, Fig. 2F). They also consisted of increasing proportions of INSS4 tumors, which are metastatic and aggressive (Brodeur et al, J Clin Oncol. 1993;11(8):1466-77), at the expense of INSS1-3 tumors usually associated with a good prognosis (Fig. 2G). Importantly, the positive correlation between SA-c1 gene expression and better prognosis was also observed when focusing on either INSS1-3 or INSS4 tumors (Fig. 9A, B). Remarkably, the expression levels of the SA-c1 module correlated to patient prognosis more strongly than the INSS classification. For instance, the group of INSS4 samples from the quartile Q1 presented a higher EFS probability than the groups of INSS1-3 samples from the quartiles Q3 and Q4 (Fig. 9A, B). We further sub-divided the SEQC cohort based on the progression of the disease, including within the category "progression" the tumors that did not respond to therapy plus those that were recurrent despite an initial therapeutic response. We thereby observed an inverse correlation between disease progression and the combined expression of the SA-c1 genes (Fig. 2H). The module of SAc1 genes related to the core sympatho-adrenal program is thus strikingly predictive of NB malignancy, patient prognosis and disease progression. The SA-c1 genes might thus play a crucial role in restraining NB malignancy.

### Example 3. The expression of NXPH1 and its receptors α-NRXN1/2 associates with favorable patient prognosis and identifies NB cells with a neural crest stem cell identity.

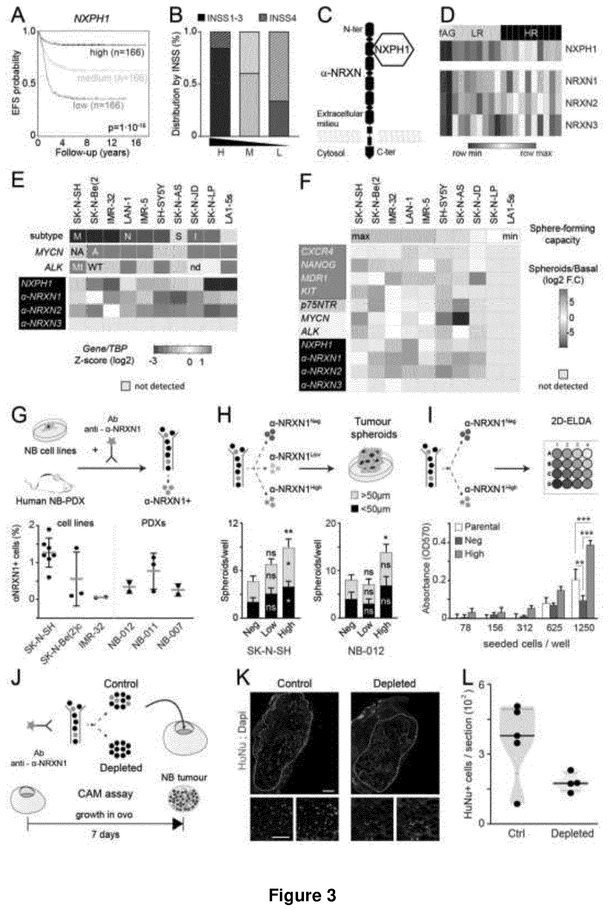

To functionally test the impact of SA-c1 genes on NB malignancy, we selected Neurexophilin-1 (NXPH1). NXPH1 was the SA-c1 gene with the second highest fold change enrichment in LR-NBs relative to HR-NBs (fc LR/HR=7.46; Fig. 2E) and its expression levels strongly correlated with favorable patient prognosis and with diminished disease progression in both the INSS1-3 and INSS4 NBs sub-groups (Fig. 3A, B and Fig.9 C-E). NXPH1 is mostly expressed in the nervous system and encodes a secreted glycoprotein that specifically binds to and modulates the activity of the α-Neurexin transmembrane receptors (α-NRXN1, 2 and 3; Fig. 3C), which are known to play key roles in synaptogenesis and neurotransmission (Missler et al. 1998 J. Neurosci. 18, 3630-8; Petrenko et al. 1996 J Neurosci. 16, 4360-4369; Reissner et al. 2014 J Biol Chem. 289, 27585-27603; Südhof et al. 2017 Cell 171(4):745-769). Interestingly, NRXN2 also came out in the list of SA-c1 genes (Fig. 2E) and both NRXN1 and NRXN2 expression levels associated with a favorable prognosis, whereas NRXN3 levels did not (Fig. 3D and Fig. 9F).

To this aim, we first characterized the expression of *NXPH1* and its *α-NRXN1*/*2*/*3* receptors in a panel of 10 human NB cell lines with diverse genetic profiles and morphological properties (Fig. 3E; Boeva et al., 2017 Nat. Genet. 49, 1408-1413; Walton et al., 2004 Neoplasia 6, 838-845). In basal culture conditions *NXPH1* and *α-NRXN1*/*2* were expressed weakly, while the expression of *α-NRXN3* transcripts was below the threshold of detection (Fig. 3E). No obvious correlation was evident between these transcripts and the *MYCN* amplification status or *ALK* mutation (Fig. 3E). Growing NB cell lines in restrictive culture conditions for 5 weeks *in vitro* can lead to the formation of spheroids that are progressively enriched in cells presenting stem cell-like characteristics and a more specific NCC identity (Hansford et al., 2007 Cancer Res 67, 11234-11243; Ikegaki et al., 2013 Proc Natl Acad Sci U S A 110, 6097-6102; Takenobu et al., 2010 Oncogene 30, 97-105). Following this protocol, most of the NB cell lines (8/10) showed the capacity to grow as stem cell-enriched spheroids and presented with an increase in the transcripts encoding the stereotypic stem cell markers *CXCR4, NANOG* and *KIT,* the NCC stem cell-specific marker *p75NTR* and the *MDR1* gene associated with chemotherapy resistance (Fig. 3F; Foster et al., 2018 Biomedicines 6 (1); Hirschmann-Jax et al., 2004 Proc Natl Acad Sci U S A 101, 14228-14233; Jiang et al., 2009 Stem Cells Dev 18(7):1059-70). Remarkably, *NXPH1* and *α-NRXN1*/*2* levels increased in all the NB cell lines harbouring a sphere-forming capacity (Fig. 3F). This finding reveals a strong positive correlation between the expression of *NXPH1* and *α-NRXN1*/*2* in NB cells, and the acquisition of a NCC stem cell identity.

Using a fluorescence-conjugated antibody recognizing the extracellular region of human α-NRXN1, we identified a small subpopulation of α-NRXN1⁺ cells (less than 1.5%) within the 3 human NB cell lines that showed the highest sphere-forming capacity and in cells dissociated from 3 different NB patient-derived xenografts (PDX; Fig. 3G). Purifying α-NRXN1^{high} cells from the SK-N-SH cell line or from the PDX NB-012 by FACS and growing them in restrictive culture conditions for a week revealed their increased spheroid-forming capacity compared to both α-NRXN1^{low} and α-NRXN1⁻ cells (Fig. 3H). In addition, α-NRXN1^{high} cells harboured an enhanced proliferative ability when seeded under conditions of extreme dilution, relative to purified α-NRXN1⁻ cells or unsorted SK-N-SH cells (Fig. 3I). To assess the importance of α-NRXN1⁺ cells *in vivo,* we compared the growth potential of SK-N-SH cells deprived of their α-NRXN1 ⁺ cell subpopulation with that of non-deprived cells using a chick chorio-allantoid membrane (CAM) assay (Fig. 3J). After 7 days of incubation *in ovo,* the number of cells quantified per tumour section was decreased by ∼50% for the α-NRXN1⁺-deprived cells relative to their control (Fig. 3K, L), thus revealing the requirement of α-NRXN1⁺ cells to support NB tumour growth *in vivo.* Together, our findings establish a correlation between *NXPH1*/*α-NRXN1* expression and the tumourigenic potential of NB cells, arguing that NXPH1/α-NRXN1 signaling could control NB growth and/or aggressiveness.

### Example 4. NXPH1/α-NRXN signaling stimulates NB growth

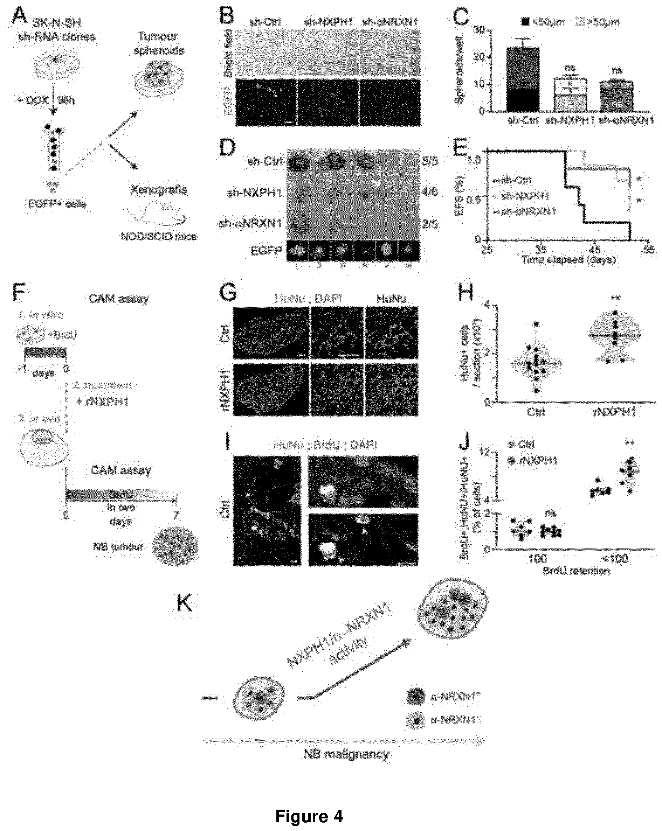

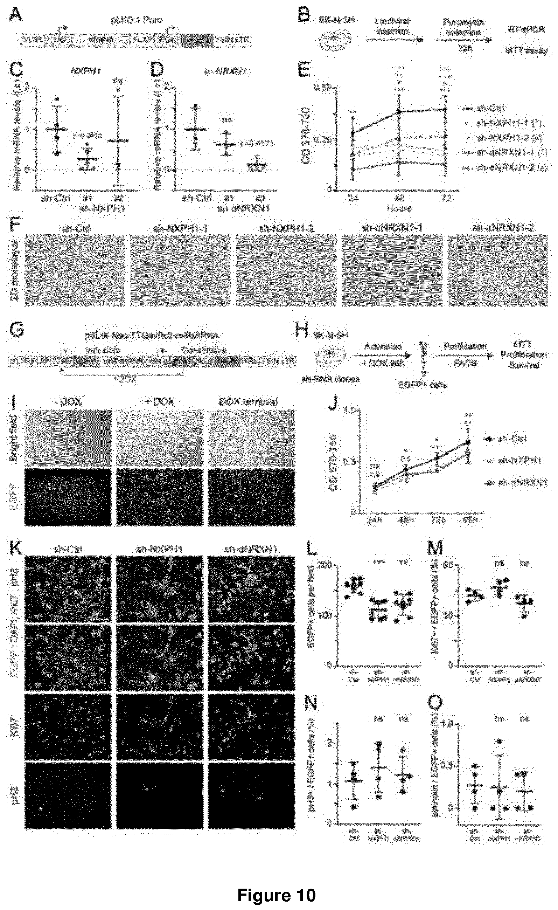

To study whether NXPH1/α-NRXN1 signaling regulates NB growth, we generated stable clones of SK-N-SH cells that constitutively express sh-RNAs targeting either *NXPH1* or *α-NRXN1* (Fig. 10A-D). Both *NXPH1* and *α-NRXN1* knockdowns severely decreased cell viability over a 3 days-period *in vitro* and caused a complete growth arrest after one week (Fig. 10E, F). To circumvent this effect, we generated clones whose sh-RNA and eGFP production was only induced upon doxycycline treatment (Fig. 10G). Using eGFP⁺ FACS-purified cells pre-treated with doxycyline for 96 hours, we observed that the growth arrest caused by both *NXPH1* and *NRXN1* knockdown was minimized in the inducible sh-NXPH1 and sh-αNRXN1 clones when grown in basal culture conditions (Fig. 10H-O). These sh-NXPH1 and sh-αNRXN1 cells had however a diminished sphere-forming capacity when grown in restrictive culture conditions (Fig. 4A-C). We next tested the effect of these inducible sh-NXPH1 and sh-αNRXN1 clones on NB growth *in vivo,* by xenografting FACS-purified, doxycyline-treated cells into the flanks of immuno-compromised NOD/SCID mice that received doxycycline *ad libitum* (Fig. 4A). The sh-NXPH1 and sh-αNRXN1 cells produced fewer and smaller tumours than their control and caused a marked delay in the time required for their detection (Fig. 4D, E). Thus, inhibiting NXPH1/α-NRXN1 signaling strongly impairs NB tumour formation *in vivo.*

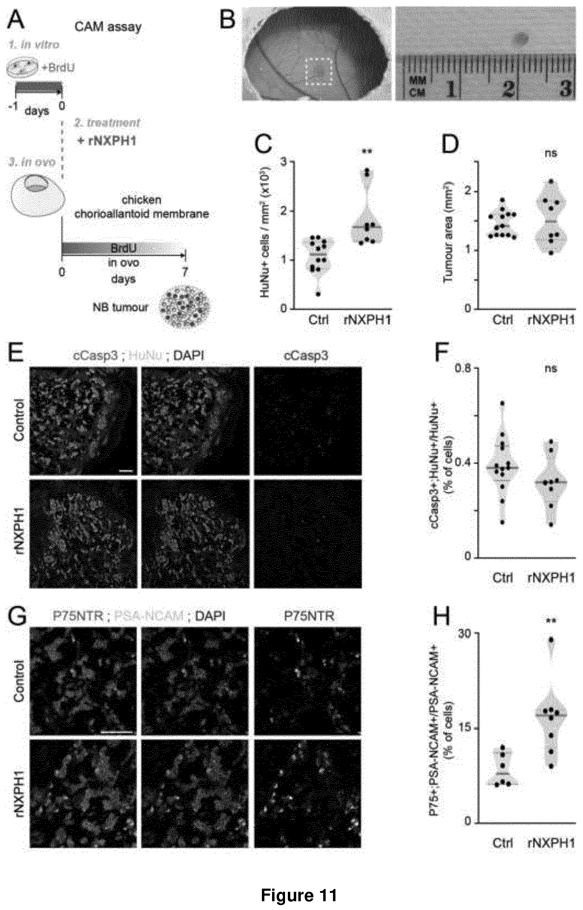

We next wondered whether stimulating NXPH1/α-NRXN1 signaling would be sufficient to increase the growth of NB tumours. To test this idea, parental SK-N-SH cells were embedded in Matrigel in the presence or absence of recombinant human NXPH1 (rNXPH1, 10 µg/ml) and seeded onto the richly vascularised CAM of 10 days-old chicken embryos (Fig. 4F). After 7 days, we found that rNXPH1 treatment increased the number of NB cells per tumour section by 62% (Fig. 4G, H and 11A-D). As SK-N-SH cells were incubated with BrdU for 24 hours *in vitro* before seeding onto the CAM, we could moreover determine that rNPHX1 addition increased the fraction of NB cells that actively cycled during the incubation *in ovo* (BrdU-ir<100%; Fig. 4I, J). By contrast, rNPHX1 treatment did not affect the proportion of NB cells that became quiescent or cycled very slowly during that period (BrdU-ir=100; Fig. 4I, J), nor apoptosis (Fig. 11E, F). Interestingly, the enhanced proliferation rate caused by rNPHX1 was associated with a 2-fold increase in the proportion of NB cells expressing the neural crest stem cell marker p75/NTR (Fig. 11G, H). Together, these data reveal that NXPH1/α-NRXN1 signaling is necessary and sufficient for NB tumour growth *in vivo* (Fig. 4K).

### Example 5. NXPH1/α-NRXN signaling inhibits the metastatic potential of NBs.

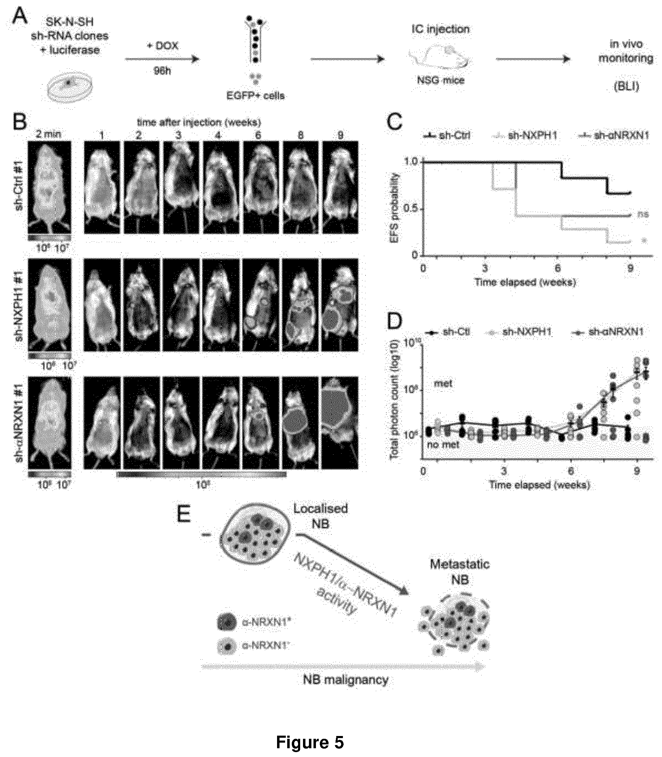

Finally, we set out to define whether NXPH1/α-NRXN1 signaling regulates the metastatic potential of NB cells *in vivo.* To this end, we generated clones of SK-N-SH cells carrying a constitutively-expressed luciferase cassette in addition to the doxycycline-inducible sh-RNAs and eGFP. FACS-purified, doxycycline-treated cells from the sh-NXPH1, sh-αNRXN1 and sh-Ctrl clones were injected into the left cardiac ventricle of immunodeficient NOD-SCID gamma (NSG) mice and their dissemination and metastasis growth was monitored non-invasively by bioluminescence (BLI) over 9 weeks (Fig. 5A). A few minutes after injection, cells from the sh-Ctrl, sh-NXPH1 and sh-αNRXN1 clones were seen disseminating within the body of the mice (Fig. 5B). Their BLI then regressed to background levels during the first 3-4 weeks, indicating that the vast majority of the cells disappeared during that period (Fig. 5B). After 9 weeks, 86% and 57% of the mice injected with sh-NXPH1 and sh-αNRXN1 cells had developed detectable metastatic tumour masses, whereas only 29% of the mice injected with the sh-Ctrl cells had (Fig. 5B, C). Moreover, the metastatic tumours developed by the sh-NXPH1 and sh-αNRXN1 cells were detected earlier and produced a photon count much higher than the sh-Ctrl cells (Fig. 5B, D). Metastases were particularly evident in the liver and bone marrow, in agreement with previous reports on the organotropism of metastatic NBs (DuBois et al., 1999 J Pediatr Hematol Oncol 21(3):181-9; Vo et al., 2014 J. Clin. Oncol. 32, 3169-3176). Thus, reducing the expression of NXPH1 or that of its α-NRXN1 receptor stimulates the metastatic potential of NB cells *in vivo.* Our latter findings therefore reveal that NXPH1/α-NRXN1 signaling represses the dissemination and metastatic potential of NBs (Fig. 5E).

## Claims

1. An activator of a gene belonging to the SA-c1 module, or a pharmaceutical composition comprising said activator in a therapeutically effective amount, for use in the treatment and/or prevention of neuroblastoma (NB) malignancies in a subject, wherein the gene is selected from the group listed in Table 1.

2. The activator or composition according to claim 1, wherein the gene is selected from the group consisting of: *SOX-6 (SOX6); Neurexophilin-1 (NXPH1); Peripherin (PRPH); Transcription Factor AP-2 Beta (TFAP2B); Ephrin type-A receptor 7 (EPHA7); Sodium channel protein type 2 subunit alpha (SCN2A); V-set and transmembrane domain-containing protein 2A (VSTM2A); Sodium channel protein type 9 subunit alpha (SCN9A); Plexin-A4 (PLXNA4); Sodium channel protein type 3 subunit alpha (SCN3A); Talin-2 (TLN2); Contactin-associated protein-like 2 (CNTNAP2); Adenylate cyclase type 1 (ADCY1); RGM domain family member B (RGMB); PH* and *SEC7 domain-containing protein 2 (PSD2).*

3. The activator or composition for use according to any one of claims 1 or 2, wherein the activator is selected from the group consisting of:
(i) an antibody or fragment thereof;
(ii) a small molecule;
(iii) an expression activator of the gene; and
(iv) an expression activator of a gene encoding a receptor, wherein the receptor binds a polypeptide encoded by *NXPH1* or *FRZB.*

4. The activator or composition for use according to any one of claims 1 to 3, wherein the pharmaceutical composition further comprises a compound for the treatment of NB malignancies in a subject.

5. The activator or composition for use according to any one of claims 1 to 4, wherein the subject is a human subject.

6. The activator or composition for use according to any one of claims 1 to 5, wherein the NB malignancy is a high-risk NB malignancy (HR-NB).

7. The activator or the composition for use according to any one of claims 1 to 6, wherein the stage of the NB malignancy is INSS4 or wherein the NB malignancy carries amplification of the oncogene MYCN.

8. The activator or composition for use according to any one of claims 1 to 7, wherein the activator inhibits the metastatic potential of the NB malignancy.

9. The activator for use according to any one of claims 1 to 8, wherein the gene is *NXPH1.*

10. An *in vitro* method for the diagnosis of NB malignancies in a subject, comprising:
(i) determining the expression levels of at least one gene selected from the group consisting of: *NXPH1; PRPH; TFAP2B; EPHA7; SCN2A; VSTM2A; SCN9A; PLXNA4; SCN3A; TLN2; CNTNAP2; ADCY1; RGMB; and PSD2* in a biological sample from the subject; and/or
(ii) determining the expression levels of *SOX6* in a biological sample from the subject; and
(ii) comparing the expression levels obtained in step (i) with a first reference expression level obtained in a fetal adrenal gland sample from a subject not suffering from NB malignancies,
wherein if the expression levels of the at least one gene listed in (i) are increased with respect to the first reference expression level, and/or the expression levels of *SOX6* are increased or decreased with respect to the first reference expression level, then the subject is suffering from an NB malignancy.

11. An *in vitro* method according to claim 10, further comprising:
(iii) comparing the expression levels obtained in step (i) with a second reference expression level obtained in a biological sample from a subject diagnosed with a LR-NB malignancy; and
(iv) comparing the expression levels obtained in step (i) with a third reference expression level obtained in a biological sample from a subject diagnosed with a HR-NB malignancy;
wherein if the expression levels of the at least one gene listed in (i) are decreased with respect to the second reference expression level and comparable to the third reference expression level, then the subject is susceptible to receive a therapy based on a gene activator according to any one of claims 1 to 9.

12. An *in vitro* method according to any one of claims 10 to 11, wherein the expression levels of *SOX6; NXPH1; PRPH; TFAP2B; EPHA7; SCN2A; VSTM2A; SCN9A; PLXNA4; SCN3A; TLN2; CNTNAP2; ADCY1; RGMB* and *PSD2* are determined.

13. An *in vitro* method according to any one of claims 11 to 12, wherein the activator is selected from the group consisting of:
(i) an antibody or fragment thereof;
(ii) a small molecule;
(iii) an expression activator of the gene; and
(iv) an expression activator of a gene encoding a receptor, wherein the receptor binds a polypeptide encoded by *NXPH1 or FRZB.*

14. An *in vitro* method according to any one of claims 10 to 13, wherein the subject is a human subject; and wherein the NB malignancy is a HR-NB malignancy.

15. An *in vitro* method according to any one of claims 10 to 14, wherein the gene is *NXPH1.*
